# EUROPEAN PATENT APPLICATION

(11) **EP 4 831 274 A1**
(43) Date of publication of application: **28.10.2026**
(21) Application number: 24907509.4
(22) Date of filing: 19.12.2024
(51) Int. Cl.: C12N 1/20, A23L 33/135, A61K 35/74, A61K 35/742, A61P 1/00, A61P 29/00, A61P 37/04

(54) **NEW STRAIN OF CLOSTRIDIUM BUTYRICUM HAVING BUTYRIC ACID PRODUCTION ABILITY**

(30) Priority: 19.12.2023 JP 2023213470
(71) Applicant: Higher Mount Co., Ltd., Gifu-shi, Gifu, 500-8441 (JP)
(72) Inventor: TAKAYAMA, Hirotaka, Gifu-shi, Gifu 500-8441 (JP)
(74) Representative: Gulde & Partner
(86) International application number: PCT/JP2024/045014
(87) International publication number: WO 2025/135125

(57) **Abstract**

The present invention aims to provide butyric acid-producing bacteria that can be used more safely as an active ingredient in intestinal regulators and the like. The present invention relates to *Clostridium butyricum* that produces n-butyric acid and has all of the following features (1) to (3):
(1) have no ability to assimilate D-xylose;
(2) have no ability to assimilate D-trehalose; and
(3) produce a lower level of formic acid in a culture liquid after 72 hours of anaerobic culture at 37°C than a NBRC13949T strain as a type strain of *Clostridium butyricum.*

## Description

### Technical Field

The present invention relates to a new strain of *Clostridium butyricum* having butyric acid production ability, a composition for intestinal regulation and the like using the same.

### Background Art

In recent years, the usefulness of butyric acid-producing bacteria has attracted attention. Butyric acid-producing bacteria, which are intestinal bacteria, decompose dietary fibers in the large intestine and produce butyric acid. Since butyric acid is used as an energy source for the large intestine to function normally, butyric acid-producing bacteria are used as an active ingredient in intestinal regulators (Non Patent Literatures 1 and 2).

*Clostridium butyricum* is a well-known example of a butyric acid-producing bacterium (Non Patent Literatures 1 to 4). *Clostridium butyricum* produces formic acid in addition to butyric acid and assimilates D-xylose and D-trehalose (Non Patent Literature 3).

### Citation List

### Non Patent Literatures

Non Patent Literature 1: Yuji Naito, "Increasing Butyric acid-Producing Bacteria can Lead to Health and Longevity: You can Know Everything About Currently Much-Talked-About Butyric acid and Butyric acid-Producing Bacteria!", Asa Publishing Co., Ltd., February 11, 2022.
Non Patent Literature 2: Akashi Eda, "Amazing Butyric acid-Producing Bacteria: Branching Point Between Those Who Will Get Sick and Those Who Won't", GENTOSHA INC., March 25, 2022.
Non Patent Literature 3: Bergey's Manual of Systematic Bacteriology, Second Edition, Volume Three, The Firmicutes, pp. 739-742 (2009)
Non Patent Literature 4: The Intestinal Microbiology Society, "Glossary: an entry of Clostridium butyricum", [online], [searched on August 5, 2023], Internet <URL: https://bifidus-fund.jp/keyword/kw002.shtml>
Non Patent Literature 5: International Chemical Safety Cards (ICSCs) Database, search results for "formic acid", [online], [searched on November 1, 2023], Internet <URL: https://www.ilo.org/dyn/icsc/showcard.listCards3>
Non Patent Literature 6: International Chemical Safety Cards (ICSC) Database, search results for "acetone", [online], [searched on November 1, 2023], Internet <URL: https://www.ilo.org/dyn/icsc/showcard.listCards3>
Non Patent Literature 7: Journal of Intestinal Microbiota, 31:15-22, 2017

### Summary of Invention

### Problems to be solved by the invention

However, formic acid is known to possess toxicity that may cause abdominal pain, stomach cramps, and diarrhea when ingested orally (Non Patent Literature 5).

In addition, acetone is also known to possess toxicity that may cause nausea and vomiting when ingested orally (Non Patent Literature 6).

Therefore, the present inventor sets an object to provide butyric acid-producing bacteria that produce low levels of formic acid and acetone and can be used more safely as an active ingredient in intestinal regulators and the like.

### Means for solution of the problems

As a result of earnest studies for this object, the present inventor found that a *Clostridium butyricum* SIID29215-B6 strain (Accession number: NITE BP-03916), isolated during the process of producing food raw materials by fermenting organically grown domestic soybeans, is (1) a novel strain with physiological and biochemical properties different from conventional *Clostridium butyricum* (the SIID29215-B6 strain does not assimilate D-xylose and D-trehalose), and (2) produces lower levels of formic acid and acetone than a type strain of *Clostridium butyricum* (NBRC13949T strain). The present invention was made based on these findings.

Specifically, the present invention relates to the following [1] to [19].
[1] *Clostridium butyricum* that produces n-butyric acid and has all of the following features (1) to (3):
   (1) have no ability to assimilate D-xylose;
   (2) have no ability to assimilate D-trehalose; and
   (3) produce a lower level of formic acid in a culture liquid after 72 hours of anaerobic culture at 37°C than a NBRC13949T strain as a type strain of *Clostridium butyricum*.
[2] A *Clostridium butyricum* SIID29215-B6 strain (Accession number: NITE BP-03916) or a mutant strain thereof, wherein the mutant strain produces n-butyric acid and has all of the following features (1) to (3):
   (1) have no ability to assimilate D-xylose;
   (2) have no ability to assimilate D-trehalose; and
   (3) produce a lower level of formic acid in a culture liquid after 72 hours of anaerobic culture at 37°C than a NBRC13949T strain as a type strain of *Clostridium butyricum*.
[3] A *Clostridium butyricum* SIID29215-B6 strain (Accession number: NITE BP-03916).
[4] A *Clostridium butyricum* SIID49520-01-B1 strain (Accession number: NITE BP-04190) or a mutant strain thereof, wherein the mutant strain produces n-butyric acid and has all of the following features (1) to (3):
   (1) have no ability to assimilate D-xylose;
   (2) have no ability to assimilate D-trehalose; and
   (3) produce a lower level of formic acid in a culture liquid after 72 hours of anaerobic culture at 37°C than a NBRC13949T strain as a type strain of *Clostridium butyricum*.
[5] A *Clostridium butyricum* SIID49520-01-B1 strain (Accession number: NITE BP-04190).
[6] A *Clostridium butyricum* SIID 50030-B1 strain (Accession number: NITE BP-04191) or a mutant strain thereof, wherein the mutant strain produces n-butyric acid and has all of the following features (1) to (3):
   (1) have no ability to assimilate D-xylose;
   (2) have no ability to assimilate D-trehalose; and
   (3) produce a lower level of formic acid in a culture liquid after 72 hours of anaerobic culture at 37°C than a NBRC13949T strain as a type strain of *Clostridium butyricum*.
[7] A *Clostridium butyricum* SIID 50030-B1 strain (Accession number: NITE BP-04191).
[8] A composition for intestinal regulation comprising, as an active ingredient, bacterial cells, spores, or a culture liquid of the *Clostridium butyricum* according to any one of the above [1] to [7].
[9] A composition for growth inhibition of *Clostridioides difficile* comprising, as an active ingredient, bacterial cells, spores, or a culture liquid of the *Clostridium butyricum* according to any one of the above [1] to [7].
[10] A composition for anti-inflammatory use comprising, as an active ingredient, bacterial cells, spores, or a culture liquid of the *Clostridium butyricum* according to any one of the above items [1] to [7].
[11] A composition for induction of regulatory T cells comprising, as an active ingredient, bacterial cells, spores, or a culture liquid of the *Clostridium butyricum* according to any one of the above [1] to [7].
[12] The composition according to the above [8], which is in the form of a pharmaceutical product.
[13] The composition according to the above [9], which is in the form of a pharmaceutical product.
[14] The composition according to the above [10], which is in the form of a pharmaceutical product.
[15] The composition according to the above [11], which is in the form of a pharmaceutical product.
[16] The composition according to the above [8], which is in the form of a food or beverage product.
[17] The composition according to the above [9], which is in the form of a food or beverage product.
[18] The composition according to the above [10], which is in the form of a food or beverage product.
[19] The composition according to the above [11], which is in the form of a food or beverage product.

### Effects of Invention

As will be shown in Examples 2 and 3 below, the strains of *Clostridium butyricum* of the present invention produce lower levels of formic acid and acetone than the conventional strain. In addition, as will be shown in Examples 8 and 10 below, the strains of *Clostridium butyricum* of the present invention produce a lower level of formic acid than the conventional strain. Therefore, the strains of the present invention are each useful as an active ingredient in products utilizing butyric acid-producing bacteria (such as intestinal regulators).

### Brief Description of Drawings

Fig. 1 shows a simplified molecular phylogenetic tree of *Clostridium butyricum* SIID29215-B6 strain (Accession number: NITE BP-03916) based on partial 16S rDNA nucleotide sequences.
Fig. 2 shows a simplified molecular phylogenetic tree of *Clostridium butyricum* SIID49520-01-B1 strain (Accession number: NITE BP-04190) based on partial 16S rDNA nucleotide sequences.
Fig. 3 shows a simplified molecular phylogenetic tree of *Clostridium butyricum* SIID 50030-B1 strain (Accession number: NITE BP-04191) based on partial 16S rDNA nucleotide sequences.

### Description of Embodiments

Strains of the present invention are of *Clostridium butyricum*.

*Clostridium butyricum* is a type species of the genus *Clostridium*, as presented in Bergey's Manual of Systematic Bacteriology, Second Edition, Volume Three, The Firmicutes, pp. 739-742 (2009) (Non Patent Literature 3) and on the website of The Intestinal Microbiology Society, Public Interest Incorporated Foundation ("Glossary: an entry of Clostridium butyricum", URL: https://bifidus-fund.jp/keyword/kw002.shtml) (Non Patent Literature 4).

A strain according to a preferred embodiment of the present invention is internationally deposited with Incorporated Administrative Agency, National Institute of Technology and Evaluation (NITE) Patent Microorganisms Depositary, which is International Depositary Authority under the provisions of the Budapest Convention, (Room 122, 2-5-8 Kazusa-Kamatari, Kisarazu City, Chiba Prefecture, Japan 292-0818) (Indication of identification: SIID29215-B6. Accession number: NITE BP-03916. Deposit date (Accession date): June 15, 2023).

A strain according to another preferred embodiment of the present invention is internationally deposited with NITE Patent Microorganisms Depositary, which is International Depositary Authority under the provisions of the Budapest Convention, (Room 122, 2-5-8 Kazusa-Kamatari, Kisarazu City, Chiba Prefecture, Japan 292-0818) (Indication of identification: SIID49520-01-B1. Accession number: NITE BP-04190. Deposit date (Accession date): October 29, 2024).

A strain according to another preferred embodiment of the present invention is internationally deposited with NITE Patent Microorganisms Depositary, which is International Depositary Authority under the provisions of the Budapest Convention, (Room 122, 2-5-8 Kazusa-Kamatari, Kisarazu City, Chiba Prefecture, Japan 292-0818) (Indication of identification: SIID 50030-B1. Accession number: NITE BP-04191. Deposit date (Accession date): October 29, 2024).

### [Features of Strains of Present Invention]

Hereinafter, features of the strains of the present invention will be described below.

### (1) n-Butyric acid Production

The strains of the present invention produce n-butyric acid.

The level of n-butyric acid production can be measured by subjecting a culture filtrate of a strain to an organic acid analysis (for example, high-performance liquid chromatography).

### (2) Sugar Assimilation

The strains of the present invention have one or preferably all two of the following features (1) to (2) regarding sugar assimilation ability (an ability to assimilate sugars as a nutrient source).
(1) have no ability to assimilate D-xylose; and
(2) have no ability to assimilate D-trehalose.

The sugar assimilation ability can be determined using a commercially available microbial identification test kit (for example, API20A (bioMerieux, FRA)).

Bergey's Manual of Systematic Bacteriology, Second Edition, Volume Three, The Firmicutes, pp. 739-742 (2009) (Non Patent Literature 3) states that *Clostridium butyricum* has the ability to assimilate D-xylose and D-trehalose.

As will be shown in Example 1 (morphological, physiological, and biochemical properties test) described later, the NBRC13949T strain, which is a type strain of *Clostridium butyricum*, also has the ability to assimilate D-xylose and D-trehalose.

Therefore, the strains of the present invention, which differ from the conventional strain in sugar assimilation ability, are new strains of *Clostridium butyricum*.

### (3) Formic acid Production

In a preferred embodiment, the level of formic acid production by the strain of the present invention is lower than that of the NBRC13949T strain as the type strain of *Clostridium butyricum*.

Specifically, as compared based on the level of formic acid production in a culture liquid after 72 hours of anaerobic culture at 37°C, the level of production by the strain of the present invention is lower than the level of production by the NBRC13949T strain, and preferably 12% or less of the level of production by the NBRC13949T strain.

The level of formic acid production can be measured by subjecting a culture filtrate of a strain to an organic acid analysis (for example, high-performance liquid chromatography).

### (4) Acetone Production

In a preferred embodiment, the level of acetone production by the strain of the present invention is lower than that of the NBRC13949T strain as the type strain of *Clostridium butyricum*.

Specifically, as compared based on the level of acetone production in a culture liquid after 48 hours of anaerobic culture at 37°C, the level of production by the strain of the present invention is less than the level of production by the NBRC13949T strain, and preferably 70% or less of the level of production by the NBRC13949T strain.

The level of acetone production can be measured by subjecting a culture supernatant of the strain to GC/MS measurement.

### (5) 16S rDNA and Genomic DNA

The strains of the present invention may have, as a partial 16S rDNA (gene encoding 16S rRNA) nucleotide sequence, a nucleotide sequence that has at least 99.6% or more, preferably 99.8% or more, and further preferably 99.9 % or more sequence identity with a nucleotide sequence specified with SEQ ID NO: 1, SEQ ID NO: 174, or SEQ ID NO: 175 (presented later).

Moreover, the strains of the present invention may have, as a genome sequence, a nucleotide sequence that has at least 99.6% or more, preferably 99.8% or more, and further preferably 99.9% or more sequence identity with a nucleotide sequence specified with any of SEQ ID NO: 2 to SEQ ID NO: 173 presented later.

### (6) Spore-Forming Capacity

In a preferred embodiment, the strain of the present invention has spore-forming capacity. In this embodiment, the spores of the strain of the present invention are usable as an active ingredient for products utilizing butyric acid-producing bacteria (such as intestinal regulators).

### (7) Other Features

In addition to the above features (1) to (6), the strains of the present invention may have one of more of the features possessed by *Clostridium butyricum* SIID29215-B6 strain (Accession number: NITE BP-03916) and shown in Examples described below (for example, heat resistance, growth inhibition of *Clostridioides difficile*, and so on).

Moreover, the strains of the present invention may have one or more of the features possessed by *Clostridium butyricum* SIID49520-01-B1 strain (Accession number: NITE BP-04190) or *Clostridium butyricum* SIID 50030-B1 strain (Accession number: NITE BP-04191) and shown in Examples described later.

In a preferred embodiment, the strain of the present invention is *Clostridium butyricum* SIID29215-B6 strain (Accession number: NITE BP-03916) or a mutant strain thereof. This mutant strain produces n-butyric acid and has all of the following features (1) to (3):
(1) have no ability to assimilate D-xylose;
(2) have no ability to assimilate D-trehalose; and
(3) produce a lower level of formic acid in a culture liquid after 72 hours of anaerobic culture at 37°C than the NBRC13949T strain as the type strain of *Clostridium butyricum*.

In a preferred embodiment, the strain of the present invention is a mutant strain of *Clostridium butyricum* SIID49520-01-B1 strain (Accession number: NITE BP-04190). This mutant strain produces n-butyric acid and has all of the following features (1) to (3):
(1) have no ability to assimilate D-xylose;
(2) have no ability to assimilate D-trehalose; and
(3) produce a lower level of formic acid in a culture liquid after 72 hours of anaerobic culture at 37°C than the NBRC13949T strain as the type strain of *Clostridium butyricum*.

In another preferred embodiment, the strain of the present invention is a mutant strain of *Clostridium butyricum* SIID 50030-B1 strain (Accession number: NITE BP-04191). This mutant strain produces n-butyric acid and has all of the following features (1) to (3):
(1) have no ability to assimilate D-xylose;
(2) have no ability to assimilate D-trehalose; and
(3) produce a lower level of formic acid in a culture liquid after 72 hours of anaerobic culture at 37°C than the NBRC13949T strain as the type strain of *Clostridium butyricum*.

The aforementioned mutant strain of each of the deposited strains is one whose physical and chemical properties are modified by a method including inducing a mutation in the deposited strain by a treatment such as drug treatment and/or ultraviolet irradiation and then screening, a spontaneous mutation (for example, a spontaneous mutation that occurs during repeated subculturing), a morphological mutation, or a genetic engineering technique such as transfection, and which produces n-butyric acid and has all of the following features (1) to (3):
(1) have no ability to assimilate D-xylose;
(2) have no ability to assimilate D-trehalose; and
(3) produce a lower level of formic acid in a culture liquid after 72 hours of anaerobic culture at 37°C than the NBRC13949T strain as the type strain of *Clostridium butyricum*.

In addition, the mutant strain of the deposited strain may have, as a partial 16S rDNA nucleotide sequence in the deposited strain, a nucleotide sequence that has at least 99.6% or more, preferably 99.8% or more, and further preferably 99.9% or more sequence identity with the nucleotide sequence specified with SEQ ID NO: 1, SEQ ID NO: 174, or SEQ ID NO: 175 (presented later).

Here, the *Clostridium butyricum* SIID29215-B6 strain (Accession number: NITE BP-03916) may be referred to as "butyric acid-producing bacteria UniRAQU strain".

### [Culture Conditions for Strains of Present Invention]

Culture of the strains of the present invention does not need any special conditions, and the strains each may be cultured under conditions commonly used for *Clostridium butyricum*.

The culture medium is preferably an anaerobic culture medium. The culture medium may be either a natural culture medium or a synthetic culture medium. In a case where the strain of the present invention is for use in food applications, a culture medium composed only of food materials and food additives may be used.

The culture can be carried out under anaerobic conditions at a temperature of, for example, 30 to 45°C (preferably 36 to 38°C). The anaerobic conditions refer to an environment hypoxic to the extent that *Clostridium butyricum* can grow. The anaerobic conditions may be established by using a sealed container, bag, or the like containing an oxygen absorber.

The culture schemes include static culture, agitated culture, tank culture, and so on.

The culture time is preferably 24 to 72 hours (including 48 hours).

Spore formation does not need any special conditions; spores may be formed by culturing the strain of the present invention under the aforementioned conditions, but it is preferable to form spores by heating at 55 to 60°C for 30 minutes.

### [Method for Obtaining Strains of Present Invention]

As the strains of the present invention, the three strains are deposited internationally as described above, and all of them can be obtained from the international depositary authority.

In addition, the strain of the present invention can also be isolated from soybeans, as in the case with the three strains deposited internationally.

*Clostridium* bacteria to which the strains of the present invention belong are soil bacteria. However, as a source of the strain of the present invention, soybeans are preferred over soil in which various types of soil bacteria reside. Although the present invention should not be limited by any particular theory, the reasons why soybeans are preferred as the source are as follows.

When soybeans are ready for harvest, their pods split open, exposing the beans inside. *Clostridium* bacteria (or spores thereof) carried up from the soil by wind and the like are attached to the beans exposed. The thin skins of soybeans are mainly composed of dietary fibers. The inside of the beans is rich in protein. The dietary fibers and the protein both are substances favorable for the growth of *Clostridium* bacteria. Therefore, there is a high probability that *Clostridium* bacteria (or spores thereof) are attached to post-harvest soybeans (especially soybeans grown organically without use of pesticides).

The *Clostridium* bacteria can be obtained from the soybeans, for example, according to the following procedure.

The soybeans are fermented with water, honey, and indigestible dextrin in an aerobic environment at 37°C for 72 hours. After completion of the fermentation, the resulting fermented liquid and soybeans are made into a paste. This fermented soybean paste is subjected to the anaerobic conditions described in the above section titled [Culture Conditions for Strains of Present Invention].

From among the *Clostridium* bacteria obtained, *Clostridium butyricum* is identified by using sequence information on the type strain of *Clostridium butyricum* (NBRC13949T strain) (for example, the known nucleotide sequence of the 16S rDNA region). The identified *Clostridium butyricum* is further screened using the features possessed by the strain of the present invention (such as the ability to assimilate D-xylose and D-trehalose and a formic acid-producing capacity) as indicators to obtain the strain of the present invention.

### [Uses of Strains of Present Invention]

The strains of the present invention can be used in the same applications as the known butyric acid-producing bacteria (particularly *Clostridium butyricum*), and can be used, for example, as active ingredients in intestinal regulators, anti-inflammatory agents, and regulatory T cell inducers.

### [Composition for Intestinal Regulation]

The butyric acid-producing bacteria are used as an active ingredient for intestinal regulators because the butyric acid-producing bacteria can maintain a normal intestinal flora through butyric acid production (Non Patent Literatures 1 and 2). The strains of the present invention have butyric acid production ability (Examples 2, 8, and 10 described later). Accordingly, one aspect of the present invention is a composition for intestinal regulation comprising, as an active ingredient, bacterial cells, spores, or a culture liquid of the strain of the present invention (hereinafter also referred to as "bacterial cells or the like of the present invention").

The active ingredient may be either bacterial cells or spores or may be a combination of bacterial cells and spores. The culture liquid may contain either bacterial cells or spores or may contain both bacterial cells and spores.

The phrase "comprising, as an active ingredient" means that the composition contains the bacterial cells or the like of the present invention in a sufficient amount (effective amount) to exert a desired intestinal regulating effect (effect of regulating an intestinal environment).

The content of the strain of the present invention can be appropriately set considering the form of a composition (such as a food or beverage or a pharmaceutical) and others. For example, in the case of a food for human consumption, the content of "bacterial cells" (number of bacteria) as an active ingredient can be set in the same manner as the number of butyric acid-producing bacteria contained in general intestinal regulators. For example, the content of bacterial cells in a food for human consumption can be set so that the daily intake of bacterial cells is preferably 300 million to 2.5 billion and more preferably 1 billion to 2 billion.

The composition for intestinal regulation may contain one or more optional ingredients, as long as they will not impair the action of the active ingredient. The optional ingredients may be selected depending on the form and the like of the composition.

The optional ingredients may be additives for foods and/or beverages or pharmaceuticals. The optional ingredients are publicly known substances, and can be readily obtained on the market or can be prepared. One type of optional ingredient may be used alone or two or more types of optional ingredients may be used in combination. The content of the optional ingredient may be set as appropriate depending on the purpose of the formulation or the like.

The composition for intestinal regulation can be applied to a wide range of animal species without particular limitations. The target population is preferably mammals (humans and non-human mammals (for example, dogs and cats)), and more preferably humans. Moreover, the target population is not limited by sex or age.

The dosage of the composition for intestinal regulation can be appropriately set according to the age and weight of a target individual, the frequency of intake, the route of administration, and so on.

The administration interval may be set as appropriate depending on the dosage and so on, and may be once a day or several times a day.

The composition for intestinal regulation can be prepared by mixing an active ingredient (the bacterial cells or the like of the present invention) with optional ingredients (for example, additives for foods and/or beverages or pharmaceuticals).

### [Composition for Growth Inhibition of Clostridioides difficile]

It is known that taking an antibiotic can disrupt the balance of the intestinal environment, leading to the abnormal proliferation of certain bacteria and causing antibiotic-associated diarrhea (such as diarrhea or loose stools). *Clostridioides difficile* is representative causative bacteria of antibiotic-associated diarrhea.

The strains of the present invention are capable of inhibiting the growth of *Clostridioides difficile* (Example 6 describe later). Therefore, one aspect of the present invention is a composition for growth inhibition of *Clostridioides difficile* comprising, as an active ingredient, bacterial cells or the like of the present invention.

The active ingredient may be either bacterial cells or spores or may be a combination of bacterial cells and spores. The culture liquid may contain either bacterial cells or spores or may contain both bacterial cells and spores.

The phrase "comprising, as an active ingredient" means that the composition contains the bacterial cells or the like of the present invention in a sufficient amount (effective amount) to exert a desired growth inhibition effect.

The content of the strain of the present invention can be appropriately set considering the form of a composition (such as a food or beverage or a pharmaceutical) and others. For example, in the case of a food for human consumption, the content of "bacterial cells" (number of bacteria) as an active ingredient can be set in the same manner as the number of butyric acid-producing bacteria contained in general intestinal regulators. For example, the content of the bacterial cells in a food for human consumption can be set so that the daily intake of bacterial cells is preferably 300 million to 2.5 billion and more preferably 1 billion to 2 billion.

The composition for growth inhibition may contain one or more optional ingredients, as long as they will not impair the action of the active ingredient. The optional ingredients may be selected depending on the form and the like of the composition. The optional ingredients may be additives for foods and/or beverages or pharmaceuticals. The optional ingredients are publicly known substances, and can be readily obtained on the market or can be prepared. One type of optional ingredient may be used alone or two or more types of optional ingredients may be used in combination. The content of the optional ingredient may be set as appropriate depending on the purpose of the formulation or the like.

The composition for growth inhibition can be applied to a wide range of animal species without particular limitations. The target population is preferably mammals (humans and non-human mammals (for example, dogs and cats)), and more preferably humans. Moreover, the target population is not limited by sex or age.

The dosage of the composition for growth inhibition can be appropriately set according to the age and weight of a target individual, the frequency of intake, the route of administration, and so on.

The administration interval may be set as appropriate depending on the dosage and so on, and may be once a day or several times a day.

The composition for growth inhibition can be prepared by mixing an active ingredient (the bacterial cells or the like of the present invention) with optional ingredients (for example, additives for foods and/or beverages or pharmaceuticals).

### [Composition for Anti-inflammatory use]

The butyric acid-producing bacteria are known to have anti-inflammatory effects (for example, effects of inhibiting enteritis, ulcerative colitis, and Crohn's disease) through butyric acid production (Non Patent Literature 1). The strains of the present invention have butyric acid production ability (Example 2 described below). Therefore, one aspect of the present invention is a composition for anti-inflammatory use comprising, as an active ingredient, the bacterial cells or the like of the present invention.

The active ingredient may be either bacterial cells or spores or may be a combination of bacterial cells and spores. The culture liquid may contain either bacterial cells or spores or may contain both bacterial cells and spores.

The phrase "comprising, as an active ingredient" means that the composition contains the bacterial cells or the like of the present invention in a sufficient amount (effective amount) to exert desired anti-inflammatory effects.

The content of the strain of the present invention can be appropriately set considering the form of a composition (such as a food or beverage or a pharmaceutical) and others. For example, in the case of a food for human consumption, the content of "bacterial cells" (number of bacteria) as an active ingredient can be set in the same manner as the number of butyric acid-producing bacteria contained in general intestinal regulators. For example, the content of bacterial cells in a food for human consumption can be set so that the daily intake of bacterial cells is preferably 300 million to 2.5 billion and more preferably 1 billion to 2 billion.

The composition for anti-inflammatory use may contain one or more optional ingredients, as long as they will not impair the action of the active ingredient. The optional ingredients may be selected depending on the form and the like of the composition.

The optional ingredients may be additives for foods and/or beverages or pharmaceuticals. The optional ingredients are publicly known substances, and can be readily obtained on the market or can be prepared. One type of optional ingredient may be used alone or two or more types of optional ingredients may be used in combination. The content of the optional ingredient may be set as appropriate depending on the purpose of the formulation or the like.

The composition for anti-inflammatory use can be applied to a wide range of animal species without particular limitations. The target population is preferably mammals (humans and non-human mammals (for example, dogs and cats)), and more preferably humans. Moreover, the target population is not limited by sex or age.

The dosage of the composition for anti-inflammatory use can be appropriately set according to the age and weight of a target individual, the frequency of intake, the route of administration, and so on.

The administration interval may be set as appropriate depending on the dosage and so on, and may be once a day or several times a day.

The composition for anti-inflammatory use can be prepared by mixing an active ingredient (the bacterial cells or the like of the present invention) with optional ingredients (for example, additives for foods and/or beverages or pharmaceuticals).

### [Composition for Induction of Regulatory T Cells]

The butyric acid-producing bacteria are known to induce regulatory T cells through butyric acid production (Non Patent Literatures 1 and 7). This induction includes differentiation induction into regulatory T cells and proliferation induction of regulatory T cells (Non Patent Literatures 1 and 7).

The regulatory T cells (Tregs) play a role in inhibiting an immune response against self (immune tolerance). The strains of the present invention have butyric acid production ability (Example 2 described below). Therefore, one aspect of the present invention is a composition for induction of regulatory T cells comprising, as an active ingredient, the bacterial cells or the like of the present invention.

The active ingredient may be either bacterial cells or spores or may be a combination of bacterial cells and spores. The culture liquid may contain either bacterial cells or spores or may contain both bacterial cells and spores.

The phrase "comprising, as an active ingredient" means that the composition contains the bacterial cells or the like of the present invention in a sufficient amount (effective amount) to exert a desired effect of inducing regulatory T cells.

The content of the strain of the present invention can be appropriately set considering the form of a composition (such as a food or beverage or a pharmaceutical) and others. For example, in the case of a food for human consumption, the content of "bacterial cells" (number of bacteria) as an active ingredient can be set in the same manner as the number of butyric acid-producing bacteria contained in general intestinal regulators. For example, the content of the bacterial cells in a food for human consumption can be set so that the daily intake of bacterial cells is preferably 300 million to 2.5 billion and more preferably 1 billion to 2 billion.

The composition for induction of regulatory T cells may contain one or more optional ingredients, as long as they will not impair the action of the active ingredient. The optional ingredients may be selected depending on the form and the like of the composition.

The optional ingredients may be additives for foods and/or beverages or pharmaceuticals. The optional ingredients are publicly known substances, and can be readily obtained on the market or can be prepared. One type of optional ingredients may be used alone or two or more types of optional ingredients may be used in combination. The content of the optional ingredient may be set as appropriate depending on the purpose of the formulation or the like.

The composition for induction of regulatory T cells can be applied to a wide range of animal species without particular limitations. The target population is preferably mammals (humans and non-human mammals (for example, dogs and cats)), and more preferably humans. Moreover, the target population is not limited by sex or age.

The dosage of the composition for induction of regulatory T cells can be appropriately set according to the age and weight of a target individual, the frequency of intake, the route of administration, and so on.

The administration interval may be set as appropriate depending on the dosage and so on, and may be once a day or several times a day.

The composition for induction of regulatory T cells can be prepared by mixing an active ingredient (the bacterial cells or the like of the present invention) with optional ingredients (for example, additives for foods and/or beverages or pharmaceuticals).

### [Use Modes of Compositions Comprising Bacterial Cells or The Like of Present Invention]

The composition for intestinal regulation, the composition for growth inhibition of *Clostridioides difficile*, the composition for anti-inflammatory use, and the composition for induction of regulatory T cells described above can be used as foods and/or beverages or pharmaceuticals.

### [Food and/or Beverage]

The form of a food or beverage is not particularly limited as long as it can be taken orally. Specific examples thereof include liquid beverages, jelly drinks, and the like.

The foods and/or beverages include health foods, functional foods, nutritional supplements, foods for specified health uses, foods for sick persons, and foods and/or beverages with disease risk reduction claims, and so on.

The foods and/or beverages may be sports supplement products (including products with Informed Sport certification) and cereal products.

The foods and/or beverages may contain food additives as optional ingredients.

Additives for liquid beverages include pH adjusters, emulsifiers, stabilizers, flavorings, sweeteners, and so on. Additives for jelly drinks include gelatin, food coloring, thickening polysaccharides, and so on.

### [Pharmaceutical]

The dosage form of the pharmaceutical is preferably one that can be administered orally or rectally (for example, by enema or the like). Specific examples thereof include liquid preparations, capsules, powder preparations, and so on.

The pharmaceutical may contain pharmaceutical additives as optional ingredients. The pharmaceutical additives include excipients, stabilizers, preservatives, humectants, emulsifiers, lubricants, sweeteners, colorants, flavorings, buffers, antioxidants, pH adjusters, and so on.

### Examples

Hereinafter, the present invention will be described in more detail by using Examples, but the present invention should not be limited to these Examples.

### [Test Strain 1]

The *Clostridium butyricum* SIID29215-B6 strain (hereinafter, also referred to as "the B6 strain") was used.

The B6 strain is internationally deposited with NITE Patent Microorganisms Depositary, which is the international depositary authority under the provisions of the Budapest Convention, (Room 122, 2-5-8 Kazusa-Kamatari, Kisarazu City, Chiba Prefecture, Japan 292-0818) (Indication of identification: SIID29215-B6. Accession number: NITE BP-03916. Deposit date (Accession date): June 15, 2023).

As the type strain of *Clostridium butyricum*, *Clostridium butyricum* NBRC13949T strain (hereinafter also referred to as "the type strain") was used.

### [Example 1: Morphological, Physiological, And Biochemical Properties Test]

The B6 strain and the type strain were cultured anaerobically at 37°C for 72 hours by using an agar culture medium (GAM Broth "Nissui" (Nissui Pharmaceutical, Japan)+agar) and using AneroPouch Kenki System (Mitsubishi Gas Chemical, Japan) as an anaerobic culture kit.

The morphology of each of the B6 strain and the type strain thus cultured was observed by using an optical microscope.

Moreover, the physiological properties of the B6 strain and the type strain (catalase reaction, oxidase reaction, acid/gas production from glucose, and oxidation/fermentation (O/F) of glucose) were tested based on the method of Barrow & Feltham et al. (Cowan and Steel's Manual for the Identification of Medical Bacteria. 3rd edition. Cambridge: University Press; 1993). The results are shown in Table 1-1.

**[Table 1-1]**

| Table 1-1. Morphological Observation and Physiological Properties Tests (Part 1) | | | |
|---|---|---|---|
| Test item | | B6 strain | Type strain |
| Culture temperature | | 37°C | 37°C |
| Cell morphology | | Rod-shaped bacterium (0.8-0.9×2.0-10.0 µm) | Rod-shaped bacterium (0.7-0.9×2.0-8.0 µm) |
| Gram stainability | | + | + |
| Presence or absence of spores | | +(Subterminal) | + (Central/Subterminal) |
| Motility | | ± | + |
| Colony form | Culture medium | GAM agar culture medium | GAM agar culture medium |
| | Culture time | 24 hours | 24 hours |
| | Diameter | 4-5 mm | N/R |
| | Tone | Pale yellow | Pale yellow |
| | Shape | Irregular shapes | N/R |
| | Elevation | Lens-shaped | N/R |
| | Margin | Undulate | Undulate |
| | Surface shape, etc. | Smooth | N/R |
| | Transparency | Opaque | N/R |
| | Consistency | Butter-like | N/R |
| Growth temperature test | 30°C | + | + |
| | 45°C | + | - |
| Catalase reaction | | - | - |
| Oxidase reaction | | - | - |
| Acid/gas production from glucose (Acid production/Gas generation) | | -/- | -/N/R |
| O/F test (Oxidation/Fermentation) | | -/- | N/R |

| | | | |
|---|---|---|---|
| +: Positive, -: Negative, +w: Weak reaction, N/R: Not measured | | | |

The B6 strain and the type strain were cultured anaerobically at 37°C for 24 hours by using an agar culture medium (GAM Broth "Nissui" (Nissui Pharmaceutical, Japan)+agar) and using AneroPouch Kenki System (Mitsubishi Gas Chemical, Japan) as an anaerobic culture kit.

The physiological and biochemical properties of the B6 strain and the type strain thus cultured were identified by using an anaerobic bacterial biochemical identification kit (API20A (bioMerieux, FRA)). The results are shown in Table 1-2.

**[Table 1-2]**

| Table 1-2. Physiological and Biochemical Properties Test (Part 2) | | | | | | | |
|---|---|---|---|---|---|---|---|
| Test item | Reaction/Enzyme | B6 strain | Type strain | Test item | Reaction/Enzyme | B6 strain | Type strain |
| IND | Indol Production* | - | - | GLY | Glycerin** | - | - |
| URE | Urease* | - | - | CEL | D-Cellobiose** | + | + |
| GLU | Glucose** | + | + | MNE | D-Mannose** | + | + |
| MAN | D-Mannitol** | - | - | MLZ | D-Melezitose** | - | + |
| LAC | Lactose** | + | + | RAF | D-Raffinose** | + | + |
| SAC | White Sugar** | + | + | SOR | D-Sorbitol** | - | - |
| MAL | Maltose** | + | + | RHA | L-Rhamnose** | - | - |
| SAL | Salicin** | + | + | TRE | D-Trehalose** | - | + |
| XYL | D-Xylose** | - | + | | | | |
| ARA | L-Arabinose** | + | + | | | | |
| GEL | Gelatin Hydrolysis* | - | - | | | | |
| ESC | Esculin Hydrolysis* | + | + | | | | |

| | | | | | | | |
|---|---|---|---|---|---|---|---|
| *Biochemistry test,**Oxidation test +: positive, -: negative | | | | | | | |

In addition, additional physiological and biochemical properties of the B6 strain were tested by using an API ZYM kit (bioMerieux, FRA). The results are shown in Table 1-3.

**[Table 1-3]**

| Table 1-3. Physiological and Biochemical Properties Test (Part 3) | |
|---|---|
| Test item | B6 strain |
| Alkaline phosphatase | - |
| Esterase (C4) | + |
| Esterase lipase (C8) | - |
| Lipase (C14) | - |
| Leucine arylamidase | - |
| Valine arylamidase | - |
| Cystine arylamidase | - |
| Trypsin | - |
| Chymotrypsin | - |
| Acid phosphatase | +w |
| Naphthol-AS-BI-phosphohydrolase | +w |
| α-Galactosidase | +w |
| β-Galactosidase | - |
| β-Glucuronidase | - |
| α-Glucosidase | + |
| β-Glucosidase | - |
| N-acetyl-β-glucosaminidase | - |
| α-Mannosidase | - |
| α-Fucosidase | - |

| | |
|---|---|
| +: Positive, -: Negative, +w: Weak reaction | |

The B6 strain did not have the ability to assimilate D-xylose and D-trehalose (Table 1-2).

On the other hand, the type strain had the ability to assimilate D-xylose and D-trehalose.

Therefore, the B6 strain was determined to be a strain different from the type strain.

### [Example 2: Organic Acid-Producing Capacity Test]

The B6 strain and the type strain were cultured anaerobically at 37°C for 72 hours by using a liquid culture medium (GAM Broth "Nissui" (Nissui Pharmaceutical, Japan)) and using AneroPouch Kenki System (Mitsubishi Gas Chemical, Japan) as an anaerobic culture kit (culture liquid volume: 5 mL).

The culture liquid was filtered through a membrane filter with a pore size of 0.20 µm to obtain a sample solution.

The concentrations of organic acids contained in the sample solution were measured using high-performance liquid chromatography.

The measurement conditions are as follows.
- System: Shimadzu's Organic Acid Analysis System (Shimadzu, Japan)
- Column: Shim-pack Fast-OA, 100 mm×7.8 mm ID, 3 columns connected in series were used.
- Guard column: Shim-pack Fast-OA, 10 mm×4.0 mm ID
- Eluent: 5 mmol/L p-toluenesulfonic acid
- Reaction solutions: 5 mmol/L p-toluenesulfonic acid, 100 µmol/L EDTA, 20 mmol/L Bis-Tris
- Flow rate: 0.8 mL/min
- Oven temperature: 50°C
- Detector: Electrical conductivity detector CDD-10Avp

Totally nine types of organic acids were measured, including succinic acid, lactic acid, formic acid, acetic acid, propionic acid, iso-butyric acid, n-butyric acid, iso-valeric acid, and n-valeric acid. Table 2 shows the results.

**[Table 2]**

| Table 2. Organic Acid-Producing Capacity Test (Production Level Unit: µg/mL) | | | | | | | | | |
|---|---|---|---|---|---|---|---|---|---|
| Sample name | Succinic acid | Lactic acid | Formic acid | Acetic acid | Propionic acid | iso-Butyric acid | n-Butyric acid | iso-Valeric acid | n-Valeric acid |
| Control (Culture medium only) | 173 | 131 | 36 | 181 | | | 4 | | |
| Type strain (NBRC13949T ) | 140 | 475 | 673 | 1091 | | | 1705 | | |
| B6 strain | 144 | 1610 | 75 | 678 | | | 1642 | | |

In the table, a blank cell indicates a value below a lower limit of quantification. The lower limit of quantification was 5 µg/mL for succinic acid, lactic acid, acetic acid, and propionic acid, and 10 µg/mL for formic acid, iso-butyric acid, n-butyric acid, iso-valeric acid, and n-valeric acid. The value for each organic acid is the average value of the three samples.

The B6 strain produced n-butyric acid. The level of formic acid production in the culture liquid after 72 hours of anaerobic culture at 37°C of the B6 strain was lower than that of the type strain (NBRC13949T).

### [Example 3: Acetone and Alcohol-Producing Capacity Test]

The B6 strain and the type strain were cultured anaerobically at 37°C for 48 hours by using a liquid culture medium (GAM Broth "Nissui" (Nissui Pharmaceutical, Japan)) and using AneroPouch Kenki System (Mitsubishi Gas Chemical, Japan) as an anaerobic culture kit.

The culture liquid after 24 and 48 hours of culture was dispensed in sterile tubes. A supernatant (10 mL or more) obtained by centrifugation of the culture liquid was filtered through a 0.2 µm cellulose acetate filter (sterilized). This filtrate refrigerated at 4°C was used as a sample.

Approximately 1 g of the sample was placed in a volumetric flask, diluted to 10 mL with methanol, agitated with a shaker for 1 hour, and then allowed to stand for 3 hours. After that, 1 µL of the supernatant was subjected to GC/MS measurement.

The measurement conditions are as follows.
- CG/MS: Agilent Technologies, 6890N/5973 inert
- Column: HP-1 (0.25 mmφ×30 m, df = 1.00 µm)
- Column temperature: 40°C (3 min)→10°C/min→100°C→20°C/min→300°C (10 min)
- Column pressure: Constant flow rate mode (51 kPa, Vac)
- Column flow rate: 1 mL/min (He)
- Inlet temperature: 250°C
- Injection volume: 1 µL
- Injection method: Split (10:1)
- Detector: MS
- Ion source temperature: 230°C
- Ionization method: EI (70 eV)
- Scan range: SIM (m/z: 31, 41, 43, 45, 58, 59, 2.0 to 3.5 min)
   : SIM (m/z: 41, 43, 45, 56, 59, 74, 3.5 to 29.0 min)
- Gain: 976 V

A pure preparation of each of components shown in Table 3 was diluted with methanol to prepare a standard solution at a certain concentration. Then, 1 µL of each standard solution was subjected to the measurement in the same manner as the sample described above, calibration curves for the respective quantification target components were created based on the peak areas of the base or molecular ions in the mass chromatograms and the prepared concentrations, and the content of each component per gram of the sample was determined. Table 3 shows the results.

**[Table 3]**

| Table 3. Acetone and Alcohol-Producing Capacity Test | | | | |
|---|---|---|---|---|
| Component | Content (µg/g) | | | |
| Name | Type strain | Type strain | B6 strain | B6 strain |
| | After 24 hours culture | After 48 hours culture | After 24 hours culture | After 48 hours culture |
| Ethanol | 270 | 280 | 190 | 180 |
| Acetone | 1.7 | 4.0 | 2.8 | 2.7 |
| Isopropanol | < 0.1 | < 0.1 | < 0.1 | < 0.1 |
| tert-Butanol | 0.43 | 0.25 | 0.23 | 0.22 |
| sec-Butanol | < 0.3 | < 0.3 | < 0.3 | < 0.3 |
| Isobutanol | 0.75 | 0.81 | 0.62 | 0.58 |
| n-Butanol | 4.9 | 5.1 | 5.0 | 4.8 |

### < Below Limit of Quantification

The level of acetone production in the culture liquid after 48 hours of anaerobic culture at 37°C of the B6 strain was lower than that of the type strain (NBRC13949T).

### [Example 4: Spore Formation Confirmation Test]

The B6 strain was cultured anaerobically at 37°C for 24 hours, 72 hours, 7 days and 14 days by using a liquid culture medium (GAM Broth "Nissui" (Nissui Pharmaceutical, Japan) or an agar culture medium (GAM Broth "Nissui" (Nissui Pharmaceutical, Japan)+agar) and using AneroPouch Kenki System (Mitsubishi Gas Chemical, Japan) as an anaerobic culture kit.

After the culture, the bacterial cells were Gram-stained (Favor G "Nissui" (Nissui Pharmaceutical, Japan) and observed with a microscopic (optical microscope: BX50F4 (Olympus, Japan)) to check a spore-forming capacity. The results are presented in Table 4.

**[Table 4]**

| Table 4. Cell Morphology Observation of B6 Strain | | | |
|---|---|---|---|
| Culture medium | Culture time | Spore formation | Gram stainability |
| Liquid culture medium | 24 hours | - | Positive |
| | 72 hours | - | |
| | 7 days | + | |
| | 14 days | + | |
| Agar culture medium | 24 hours | + | Positive |
| | 72 hours | + | |
| | 7 days | + | |
| | 14 days | + | |

| | | | |
|---|---|---|---|
| +: Positive, -: Negative | | | |

The B6 strain was spore-forming, Gram-positive bacteria.

### [Example 5: Heat Resistance Test]

The B6 strain was pre-cultured under anaerobic conditions at 37°C for 24 hours or 72 hours by using an agar culture medium (GAM Broth "Nissui" (Nissui Pharmaceutical, Japan)+agar) and using AneroPouch Kenki System (Mitsubishi Gas Chemical, Japan) as an anaerobic culture kit.

The culture was suspended in 10 mL of physiological saline to prepare a bacterial suspension. Then, 5 mL of the bacterial suspension was dispensed into a sterile medium-sized test tube (18×170 mm) and subjected to heat treatment in a water bath at 60°C for 30 minutes.

As a control, 5 mL of the bacterial suspension before the heat treatment was used.

The viable bacteria count before and after the heat treatment were measured according to the following conditions. The results are shown in Table 5.
- Culture medium: GAM Broth "Nissui" (Nissui Pharmaceuticals Japan) and agar
- Culture temperature: 37°C
- Culture time: 24 hours to 48 hours
- Diluent: Physiological saline
- Dilution ratio: Undiluted to 10⁵ times dilution
- Measurement method: Dilution plate method (0.1 mL surface plating; CFU method)
- Automatic diluter and plater: easySpiralDilute (Registered Trademark) (Interscience, France)
- Number of platings: 3 plates per dilution
- Other: Anaerobic culture (AneloPouch Kenki System (Mitsubishi Gas Chemical, Japan))
- Colony observation: Stereomicroscope (SMZ800N (Nikon, Japan))

**[Table 5]**

| Table 5. Heat Resistance Test (Quantitative) | | | | | | |
|---|---|---|---|---|---|---|
| Preculture time | Heat treatment | Dilution ratio | Colony-forming count (CFU) | | | Bacteria count (CFU/mL) |
| | | | Plate 1 | Plate 2 | Plate 3 | |
| 24 hours | None | 10² | 142 | 161 | 170 | 1.6×10⁵ |
| | Treated | 10² | 85 | 91 | 105 | 9.4×10⁴ |
| 72 hours | None | Undiluted | 23 | 27 | 28 | 2.6×10² |
| | Treated | Undiluted | 5 | 13 | 17 | 1.2×10² |

| | | | | | | |
|---|---|---|---|---|---|---|
| CFU: Colony-forming unit | | | | | | |

Since viable bacteria proliferated from the bacterial solution after the heat treatment, the B6 strain was determined to be heat-resistant.

### [Example 6: Test on Growth Inhibition of Clostridioides difficile]

The effect of the B6 strain against the growth of *Clostridioides difficile*, the representative causative bacteria of antibiotic-associated diarrhea (diarrhea or loose stools that occur as a side effect of antibiotics) was evaluated.

### (1) Preparation of Sterile Filtrate of Clostridium butyricum B6 Strain

The B6 strain was cultured under anaerobic conditions at 37°C for 72 hours by using a liquid culture medium (GAM Broth "Nissui" (Nissui Pharmaceutical, Japan)) and using AneroPouch Kenki System (Mitsubishi Gas Chemical, Japan) as an anaerobic culture kit. The total bacteria count in the culture liquid was measured according to the following conditions. The results are shown in Table 6-1.
- Diluent: Physiological saline
- Dilution ratio: Undiluted to 10⁴ times dilution
- Hemocytometer: Disposable hemocytometer. C-Chip (DHC-N01: Improved Neubawell model) (NanoEntek, Korea). Counting was performed at a dilution ratio yielding 100 to 1,000 cells per large square.
- Microscope: Optical microscope (BX50 (Olympus, Japan))

**[Table 6-1]**

| Table 6-1. Total Bacteria Count in B6 Strain Culture Liquid | | | | | | |
|---|---|---|---|---|---|---|
| Dilution ratio | Bacteria count | | | | | Total bacteria count (cells/ml) |
| | Square 1 | Square 2 | Square 3 | Square 4 | Average | |
| 10³ | 104 | 124 | 114 | 139 | 120.25 | 1.2×10⁹ |

This culture liquid was filtered and sterilized using a 0.2 µm cellulose acetate filter to obtain a sterile filtrate.

### (2) Preparation of Test Culture Medium

An equal-volume mixture of the sterile filtrate prepared in (1) and double-strength GAM Broth "Nissui" (Nissui Pharmaceutical, Japan) was used as a test culture medium.

GAM Broth "Nissui" (Nissui Pharmaceutical, Japan) was used as a control.

### (3) Preparation of Clostridioides difficile Bacterial Suspension

A type strain of *Clostridioides difficile*, JCM1296T, was cultured under anaerobic conditions at 37°C for 24 hours by using an agar culture medium (GAM Broth "Nissui" (Nissui Pharmaceutical, Japan)+agar) and using AneroPouch Kenki System (Mitsubishi Gas Chemical, Japan) as an anaerobic culture kit.

The culture liquid was suspended in sterile physiological saline and adjusted to a concentration equivalent to McFarland turbidity standard solution number 0.5 (McFarland No. 0.5). This suspension was counted using a hemocytometer and adjusted to a cell density of approximately 1×10⁸ cells/mL.

### (4) Culture of Clostridioides difficile

In a medium vial in which 20 mL of the test culture medium was dispensed, 20 µL of the *Clostridioides difficile* bacterial suspension prepared in (3) was inoculated and cultured statically under anaerobic conditions at 37°C using AneroPouch Kenki System (Mitsubishi Gas Chemical, Japan) as an anaerobic culture kit.

After the culture for 24 hours and 48 hours, 2 mL of the culture liquid was taken, and the bacteria count of *Clostridioides difficile* was measured according to the following conditions. The results are shown in Table 6-2.
- Culture medium: GAM Broth "Nissui" (Nissui Pharmaceutical, Japan)+agar
- Culture temperature: 37°C
- Culture time: 24 hours to 48 hours
- Diluent: Physiological saline
- Dilution ratio: Undiluted to 10⁷ times dilution
- Measurement method: Dilution plate method (0.1 mL surface plating; counting of colony-forming units (CFUs))
- Automatic diluter and plater: easySpiralDilute (Registered Trademark) (Interscience, France)
- Number of platings: 3 plates per dilution
- Other: Anaerobic culture (AneloPouch Kenki System (Mitsubishi Gas Chemical, Japan))
- Colony observation: Stereomicroscope (SMZ800N (Nikon, Japan))
- Judgment: Counting of colonies that grew on the plate at an appropriate dilution level.

**[Table 6-2]**

| Table 6-2. Bacteria Count of *Clostridioides difficile* | | | | | | |
|---|---|---|---|---|---|---|
| Culture time | Test culture medium | Dilution ratio | Colony-forming count (CFU) | | | Bacteria count (CFU/mL) |
| | | | Plate 1 | Plate 2 | Plate 3 | |
| 24 hours | Sterile filtrate of B6 strain added | 10³ | 145 | 152 | 170 | 1.6×10⁶ |
| | Control | 10⁵ | 132 | 156 | 162 | 1.5×10⁸ |
| 48 hours | Sterile filtrate of B6 strain added | Undiluted | 0 | 0 | 0 | Not detected |
| | Control | 10⁶ | 47 | 57 | 66 | 5.7×10⁸ |

| | | | | | | |
|---|---|---|---|---|---|---|
| CFU: Colony-forming unit | | | | | | |

The addition of the sterile filtrate of the B6 strain resulted in the growth inhibition of *Clostridioides difficile*. This suggests that the B6 strain produced a substance inhibiting the growth of *Clostridioides difficile*.

### [Reference Example 1: Analysis on Partial 16S rDNA Nucleotide Sequence]

A partial nucleotide sequence (SEQ ID NO: 1) of 16S rDNA (16S rRNA gene) of the B6 strain was analyzed to estimate its taxonomic assignment.

[SEQ ID NO: 1]
GACGAACGCTGGCGGCGTGCTTAACACATGCAAGTCGAGCGATGAAGCTCC
TTCGGGAGTGGATTAGCGGCGGACGGGTGAGTAACACGTGGGTAACCTGCCT
CATAGAGGGGAATAGCCTTTCGAAAGGAAGATTAATACCGCATAAGATTGTAG
TACCGCATGGTACAGCAATTAAAGGAGTAATCCGCTATGAGATGGACCCGCG
TCGCATTAGCTAGTTGGTGAGGTAACGGCTCACCAAGGCGACGATGCGTAGC
CGACCTGAGAGGGTGATCGGCCACATTGGGACTGAGACACGGCCCAGACTC
CTACGGGAGGCAGCAGTGGGGAATATTGCACAATGGGGGAAACCCTGATGC
AGCAACGCCGCGTGAGTGATGACGGTCTTCGGATTGTAAAGCTCTGTCTTTA
GGGACGATAATGACGGTACCTAAGGAGGAAGCCACGGCTAACTACGTGCCA
GCAGCCGCGGTAATACGTAGGTGGCAAGCGTTGTCCGGATTTACTGGGCGTA
AAGGGAGCGTAGGTGGATATTTAAGTGGGATGTGAAATACYCGGGCTTAACC
TGGGTGCTGCATTCCAAACTGGATATCTAGAGTGCAGGAGAGGAAAGGAGA
ATTCCTAGTGTAGCGGTGAAATGCGTAGAGATTAGGAAGAATACCAGTGGCG
AAGGCGCCTTTCTGGACTGTAACTGACACTGAGGCTCGAAAGCGTGGGGAG

CAAACAGGATTAGATACCCTGGTAGTCCACGCCGTAAACGATGAATACTAGG
TGTAGGGGTTGTCATGACCTCTGTGCCGCCGCTAACGCATTAAGTATTCCGCC
TGGGGAGTACGGTCGCAAGATTAAAACTCAAAGGAATTGACGGGGGCCCGC
ACAAGCAGCGGAGCATGTGGTTTAATTCGAAGCAACGCGAAGAACCTTACCT
AGACTTGACATCTCCTGAATTACTCTGTAATGGAGGAAGCCACTTCGGTGGC
AGGAAGACAGGTGGTGCATGGTTGTCGTCAGCTCGTGTCGTGAGATGTTGGG
TTAAGTCCCGCAACGAGCGCAACCCTTATTGTTAGTTGCTACCATTTAGTTGA
GCACTCTAGCGAGACTGCCCGGGTTAACCGGGAGGAAGGTGGGGATGACGT
CAAATCATCATGCCCCTTATGTCTAGGGCTACACACGTGCTACAATGGTCGGT
ACAATGAGATGCAACCTCGCGAGAGTGAGCAAAACTATAAAACCGATCTCAG
TTCGGATTGTAGGCTGAAACTCGCCTACATGAAGCTGGAGTTGCTAGTAATCG
CGAATCAGAATGTCGCGGTGAATACGTTCCCGGGCCTTGTACACACCGCCCG
TCACACCATGAGAGTTGGCAATACCCAAAGTTCGTGAGCTAACCGCAAGGA
GGCAGCGACCTAAGGTAGGGTCAGCGATTGGGGTGAAG

The analysis was conducted under the following conditions. The results are shown in Tables 7-1 and 7-2.
- DNA extraction: Cica geneus DNA extraction reagent ST (Kanto Chemical, Japan)
- PCR amplification: TKs Gflex DNA Polymerase (Takara Bio, Japan)
- Cycle Sequence: BigDye Terminator v3.1 Cycle Sequencing Kit (Applied Biosystems, USA)
- Used primers:
   PCR amplification: 9F, 1510R
   Sequencing (approx. 1500 bp): 9F, 515F, 1099F, 536R, 926R, 1510R
- Sequencer: ABI PRISM 3500xL Genetic Analyzer System (Applied Biosystems)
- Nucleotide sequence determination: ChromasPro 2.1 (Technelysium, AUS)
- BLAST homology search:
   Analysis software: ENKI v3.2 (TechnoSuruga Laboratory, Japan)
   Database:
      DB-BA 17.0 (TechnoSuruga Laboratory)
      International DNA sequencing database (DDBJ/ENA/GenBank)
- Simplified molecular phylogenetic tree analysis:
   Phylogenetic tree estimation: Neighbor-joining method
   Nucleotide substitution model: Kimura-2-parameter
   Reliability evaluation of tree structure: Bootstrap method (1,000 repetitions)

**[Table 7-1]**

| Table 7-1 BLAST Search Results of B6 Strain against DB-BA: Homology Rate with Top 30 Nucleotide Sequences | | | | | |
|---|---|---|---|---|---|
| | Registered Name | Strain Name | Accession No. | Homology Rate | BSL |
| | | | | | |
| 1 | *Clostridium butyricum* | JCM1391 | AB595129 | 1437/1438 (99.9%) | |
| 2 | *Clostridium punicoum* | DSM2619 | X71857 | 1404/1438 (97.6%) | |
| 3 | *Clostridium beijerinckii* | DSM791 | X68179 | 1406/1439 (97.7%) | |
| 4 | *Clostridium chromiireducens* | GCAF-1 | AY228334 | 1400/1438 (97.4%) | |
| 5 | *Clostridium saccharoperbutylacetonicum* | N1-4 | U16122 | 1389/1419 (97.9%) | |
| 6 | *Clostridium saccharobutylicum* | NCP262 | U16147 | 1382/1419 (97.4%) | |
| 7 | *Clostridium neonatate* | CCUG46077 | AF275949 | 1390/1440 (96.5%) | |
| 8 | *Clostridium paraputrificum* | JCM1293 | AB536771 | 1379/1440 (95.8%) | |
| 9 | *Clostridium vincentii* | lac-1 | X97432 | 1368/1440 (95.0%) | |
| 10 | *Clostridium chartatabidum* | DSM5482 | X71850 | 1365/1437 (95.0%) | |
| 11 | *Clostridium uliginosum* | CK55 | AJ276992 | 1355/1422 (95.3%) | |
| 12 | *Clostridium saudiense* | JCC | HG726039 | 1364/1442 (94.6%) | |
| 13 | *Clostridium sardiniense* | DSM2632 | AB161367 | 1364/1442 (94.6%) | |
| 14 | *Clostridium tertium* | JCM6289 | AB618789 | 1363/1442 (94.5%) | 1* |
| 15 | *Clostridium disporicum* | DSM5521 | Y18176 | 1364/1443 (94.5%) | 1* |
| 16 | *Clostridium sartagoforme* | DSM1292 | Y18175 | 1361/1442 (94.4%) | |
| 17 | *Clostridium colicanis* | DSM13634 | AJ420008 | 1356/1436 (94.4%) | |
| 18 | *Clostridium baratii* | ATCC27638 | X68174 | 1358/1442 (94.2%) | 1* |
| 19 | *Clostridium gasigenes* | DSM12272 | AF092548 | 1358/1442 (94.2%) | |
| 20 | *Clostridium chauvoei* | ATCC10092 | U51843 | 1355/1441 (94.0%) | 2 |
| 21 | *Clostridium quinii* | DSM6736 | X76745 | 1360/1443 (94.2%) | |
| 22 | *Clostridium moniliforme* | JCM9990 | AB540985 | 1357/1442 (94.1%) | 1* |
| 23 | *Clostridium tarantellae* | DSM3997 | FR733677 | 1351/1438 (93.9%) | 1* |
| 24 | *Clostridium septicum* | ATCC12464 | U59278 | 1351/1441 (93.8%) | 2 |
| 25 | *Clostridium budayi* | ATCC25541 | AB018183 | 1349/1441 (93.6%) | |
| 26 | *Clostridium algidicarnis* | NCFB2931 | AF127023 | 1344/1441 (93.3%) | |
| 27 | *Inconstantimicrobium porci* | WCA-383-APC-5B | MN537551 | 1343/1442 (93.1%) | |
| 28 | *Clostridium putrefaciens* | DSM1291 | AF127024 | 1343/1441 (93.2%) | |
| 29 | *Clostridium covendishii* | BL-28 | DQ196621 | 1331/1424 (93.5%) | |
| 30 | *Clostridium fungisolvens* | TW1 | LC020506 | 1344/1447 (92.9%) | |

| | | | | | |
|---|---|---|---|---|---|
| Note 1) In BSL (Bio Safety Level), Level 1* (Opportunistic pathogens) or higher is written and a blank cell means Level 1. Note 2) The underscore "_" in the strain name represents a space. Note 3) The top 11 strains in Table represent sequence data used for simplified molecular phylogenetic analysis. | | | | | |

**[Table 7-2]**

| Table 7-2 BLAST Search Results of B6 Strain against International DNA Sequencing Database: Homology Rate with Top 30 Nucleotide Sequences | | | |
|---|---|---|---|
| Registered Name | Strain Name | Accession No. | Homology Rate |
| Clostridium butyricum | 16-3 | CP053292 | 1437/1438 (99.9%) |
| Clostridium butyricum | CFSA3989 | CP033249 | 1437/1438 (99.9%) |
| Clostridium butyricum | CFSA3987 | CP033247 | 1437/1438 (99.9%) |
| Clostridium butyricum | NBRC 13949 | AP019716 | 1437/1438 (99.9%) |
| Clostridium butyricum | 4-1 | CP039705 | 1437/1438 (99.9%) |
| Clostridium butyricum | MSK | MK571204 | 1437/1438 (99.9%) |
| Clostridium butyricum | S-45-5 | CP030775 | 1437/1438 (99.9%) |
| Clostridium butyricum | TK520 | CP016332 | 1437/1438 (99.9%) |
| Clostridium butyricum | CBM588 | CP132344 | 1437/1438 (99.9%) |
| Clostridium butyricum | TOA | CP014704 | 1437/1438 (99.9%) |
| Clostridium butyricum | DKU-11 | CP128832 | 1437/1438 (99.9%) |
| Clostridium butyricum | SJ1 | CP127838 | 1437/1438 (99.9%) |
| Clostridium butyricum | JKY6D1 | CP013352 | 1437/1438 (99.9%) |
| Clostridium butyricum | KNU-L09 | CP013252 | 1437/1438 (99.9%) |
| Clostridium butyricum | LV1 | CP114763 | 1437/1438 (99.9%) |
| Clostridium butyricum | NEC4 | LN828931 | 1437/1438 (99.9%) |
| Clostridium butyricum | QXYZ514 | CP049778 | 1437/1438 (99.9%) |
| Clostridium sp. | LQ25 | CP111077 | 1437/1438 (99.9%) |
| Clostridium butyricum | CD-M-8 | OP491983 | 1437/1438 (99.9%) |
| Clostridium butyricum | CFSA-TJ-E | CP053374 | 1437/1438 (99.9%) |
| Clostridium butyricum | BUT1 | ON142182 | 1437/1438 (99.9%) |
| Clostridium sp. | BG-M-101 | OK271747 | 1437/1438 (99.9%) |
| Clostridium sp. | B2-M-100 | OK271596 | 1437/1438 (99.9%) |
| Clostridium butyricum | CBUT | CP073277 | 1437/1438 (99.9%) |
| Clostridium butyricum | DS501 | CP071447 | 1437/1438 (99.9%) |
| Clostridium butyricum | CB1 | MW447605 | 1437/1438 (99.9%) |
| Clostridium butyricum | CB TO-A | AB687551 | 1437/1438 (99.9%) |
| Clostridium butyricum | DSM 10702 | CP040626 | 1437/1438 (99.9%) |
| Clostridium butyricum | JCM 1391 | NR_113244 | 1437/1438 (99.9%) |
| rumen bacterium | R4_91_51 | EU124833 | 1437/1438 (99.9%) |

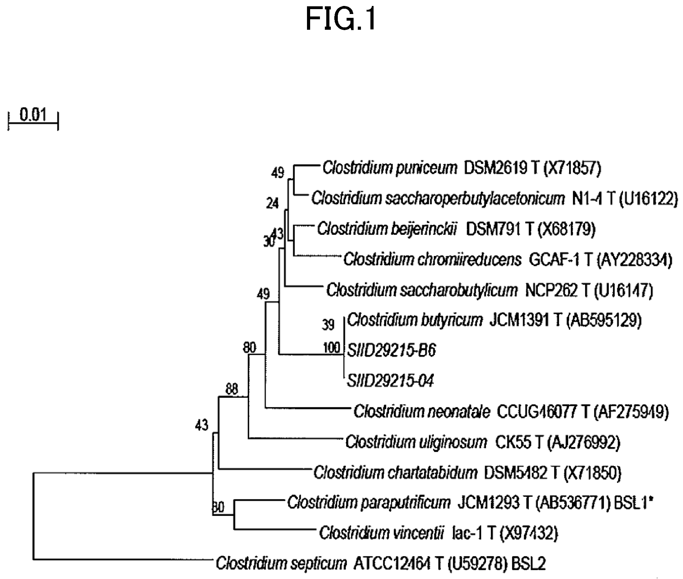

Fig. 1 shows a simplified molecular phylogenetic tree of the B6 strain based on the partial 16S rDNA nucleotide sequences. In Fig. 1, SIID29215-B6 represents the B6 strain. Meanwhile, SIID29215-04 represents the type strain *(Clostridium butyricum* NBRC13949T strain). A line on the left upper side shows a scale bar. The number located at each branching point of phylogenetic branches represents a bootstrap value, which is a values indicating the reliability of the tree structure. The suffix T of the strain name indicates the type strain (Type) of that species. BSL indicates the biosafety level (BSL1* (opportunistic pathogen) or higher).

### [Reference Example 2: in silico DDH (DNA-DNA hybridization) Analysis]

In silico DDH analysis is a method of comparing the whole genome sequences or draft genome sequences of a control strain and a comparison strain on a computer, thereby evaluating whether the two strains are of the same species or not.

In the present reference example, ANI analysis and GGDC analysis, which are different in calculation method, were conducted.

### (1) ANI (Average Nucleotide Identity) Analysis

In the ANI analysis, the genome sequence of a control strain is fragmented into 1,020 bp segments on a computer, a homology search is performed on each segment against the genome sequence of a comparison strain, and then an ANI value between the genome sequences is calculated from the average of these homology values. The ANI value is calculated by using a publicly available program ANI Calculator (http://enve-omics.ce.gatech.edu/ani/index). In the ANI analysis, the strains are determined to be of the same species if the ANI value is 95% or higher.

In the present reference example, the ANI analysis was conducted by using the B6 strain as a comparison strain and the type strain (*Clostridium butyricum* NBRC13949T strain) as a control strain, so that the obtained ANI value between the B6 strain and the type strain was 100%.

### (2) GGDC (Genome-to-Genome Distance Calculator) Analysis

In the GGDC analysis, highly homologous regions between the genome sequences of a control strain and a comparison strain are identified on a computer and the in silico DDH value is determined by calculating their pairwise distances (BMC Bioinformatics 2013; 14:60). The in silico DDH value is calculated by using a publicly available program Genome-to-Genome Distance Calculator (http://ggdc.dsmz.de/ggdc.php#). In the GGDC analysis, the strains are determined to be of the same species if the in silico DDH value is 70% or higher.

In the present reference example, the GGDC analysis was conducted by using the B6 strain as a comparison strain and the type strain (*Clostridium butyricum* NBRC13949T strain) as a control strain, so that the obtained in silico DDH value between the B6 strain and the type strain was 99.9%.

Based on the results in Reference Examples 1 and 2, the B6 strain was determined to be *Clostridium butyricum*.

Here, the B6 strain did not have the ability to assimilate D-xylose and D-trehalose (Example 1).

On the other hand, the NBRC13949T strain (the type strain of *Clostridium butyricum*) had the ability to assimilate D-xylose and D-trehalose (Example 1).

In addition, known strains of *Clostridium butyricum* are known to have the ability to assimilate D-xylose and D-trehalose (Bergey's Manual of Systematic Bacteriology, Second Edition, Volume Three, The Firmicutes, pp. 739-742 (2009) (Non Patent Literature 3)).

Therefore, the B6 strain was determined to be a strain different from the known strains of *Clostridium butyricum*, including the type strain.

### [Reference Example 3: Genome Analysis on B6 strain]

A genome analysis on the B6 strain was conducted according to the method described below.

### 1. DNA extraction

- DNA extraction and purification: NucleoSpin Plant II (MACHEREY-NAGEL, GER)

### 2. Library preparation

- Used kits: Nextera DNA Flex Library Prep Kit (Illumina, USA)
   Nextera DNA CD Indexes (Illumina)

### 3. Genome sequencing

Sequence determination was performed according to protocol included with a sequencer.
- Sequencer: iSeq 100 System (Illumina)
- Sequencing kit: iSeq 100 i1 Reagent kit (Illumina)
- Sequencing method: 2×151 bp paired-end sequencing

### 4. Data analysis

- Quality filtering: trimmomatic ver 0.39
- de novo assembly: Spades ver 3.15.4
- Correction of misassembly (polishing): pilon ver 1.24
- Calculation of coverage (depth): bowtie2 ver 2.3.5.1, samtools ver 1.10

Depth of coverage was calculated as the mean value per site by mapping the reads to the longest contig.
- Calculation of G+C content: SeqKit ver 0.12.0

The results are shown below.

| | Coverage | Genome size (Mb)^{a)} | G+C content (%)^{b}) |
|---|---|---|---|
| B6 strain | 70.5× | 3.87 | 28.6 |

a) The value indicates the sum of the lengths of contigs/scaffolds.
b) The value indicates a value calculated from the assembled nucleotide sequence.

Here, 172 contig sequences of the B6 strain thus obtained were denoted by SEQ ID NO: 2 to 173. The sequences specified with SEQ ID NO: 2 to 173 are listed in the sequence listing included in the present international application at the time of filing.

### [Reference to Deposited Biological Material]

Name of depositary authority: NITE Patent Microorganisms Depositary
Contact address: Room 122, 2-5-8 Kazusa-Kamatari, Kisarazu City, Chiba Prefecture, Japan 292-0818
Accession No: NITE BP-03916
Indication of identification: SIID29215-B6
Deposit date: June 15, 2023
Origin: Japan
Source of isolation: Isolated from a fermentation liquid containing a culture medium obtained at a food raw material manufacturing plant of HigherMount Co., Ltd. in Kaizu City, Gifu Prefecture. As the culture medium, domestically produced soybeans were used. The soybeans used were organically grown in Japan without use of pesticides.

### [Test Strain 2]

The *Clostridium butyricum* SIID49520-01-B1 strain (hereinafter also referred to as "01-B1 strain") was used.

The 01-B1 strain is internationally deposited with NITE Patent Microorganisms Depositary, International Depositary Authority under the provisions of the Budapest Convention (Room 122, 2-5-8 Kazusa-Kamatari, Kisarazu City, Chiba Prefecture, Japan 292-0818) (Indication of identification: SIID49520-01-B1. Accession number: NITE BP-04190. Deposit date (Accession date): October 29, 2024).

### [Example 7: Morphological, Physiological, And Biochemical Properties Test]

The 01-B1 strain was cultured anaerobically at 37°C for 24 hours by using an agar culture medium (AccuDia GAM Broth (Shimadzu Diagnostics Corporation, Japan)+agar) and using AneroPouch Kenki System (Mitsubishi Gas Chemical, Japan) as an anaerobic culture kit.

The morphology of the cultured 01-B1 strain was observed by using an optical microscope.

Moreover, the physiological properties of the 01-B1 strain (catalase reaction, oxidase reaction, acid/gas production from glucose, and oxidation/fermentation (O/F) of glucose) were tested based on the method of Barrow & Feltham et al. (Cowan and Steel's Manual for the Identification of Medical Bacteria. 3rd edition. Cambridge: University Press; 1993). The results are shown in Table 8-1.

The results of the type strain in Table 8-1 were obtained in Example 1.

**[Table 8-1]**

| Table 8-1. Morphological Observation and Physiological Properties Tests (Part 1) | | | |
|---|---|---|---|
| Test item | | 01-B1 strain | Type strain |
| Culture temperature | | 37°C | 37°C |
| Cell morphology | | Rod-shaped bacterium (0.9-1.1×2.0-5.0 µm) | Rod-shaped bacterium (0.7-0.9×2.0-8.0 µm) |
| Gram stainability | | + | + |
| Presence or absence of spores | | + | + (Center/Subterminal) |
| Motility | | - | + |
| Colony form | Culture medium | GAM broth agar culture medium | GAM agar culture medium |
| | Culture time | 24 hours | 24 hours |
| | Diameter | 2-5 mm | N/R |
| | Tone | Pale yellow | Pale yellow |
| | Shape | Irregular shapes | N/R |
| | Elevation | Lens-shaped | N/R |
| | Margin | Undulate | Undulate |
| | Surface shape, etc. | Smooth | N/R |
| | Transparency | Opaque | N/R |
| | Consistency | Butter-like | N/R |
| Growth temperature test | 30°C | + | + |
| | 45°C | + | - |
| Catalase reaction | | - | - |
| Oxidase reaction | | - | - |
| Acid/gas production from glucose (Acid production/Gas generation) | | -/- | -/N/R |
| O/F test (Oxidation/Fermentation) | -/- | N/R | |

| | | | |
|---|---|---|---|
| +: Positive, -: Negative, +w: Weak reaction, N/R: Not measured | | | |

The 01-B1 strain was cultured anaerobically at 37°C for 24 hours by using an agar culture medium (AccuDia GAM Broth (Shimadzu Diagnostics Corporation, Japan)+agar) and using AneroPouch Kenki System (Mitsubishi Gas Chemical, Japan) as an anaerobic culture kit.

The physiological and biochemical properties of the cultured 01-B1 strain were identified by using an anaerobic bacterial biochemical identification kit (API20A (bioMerieux, FRA)). The results are shown in Table 8-2.

The results of the type strain in Table 8-2 were obtained in Example 1.

**[Table 8-2]**

| Table 8-2. Physiological and Biochemical Properties Test (Part 2) | | | | | | | |
|---|---|---|---|---|---|---|---|
| Test item | Reactions/Enzymes | 01-B1 strain | Type strain | Test item | Reaction/Enzyme | 01-B1 strain | Type strain |
| IND | Indol Production* | - | - | GLY | Glycerin** | - | - |
| URE | Urease* | - | - | CEL | D-Cellobiose** | + | + |
| GLU | Glucose** | + | + | MNE | D-Mannose** | + | + |
| MAN | D-Mannitol** | - | - | MLZ | D-Melezitose** | - | + |
| LAC | Lactose** | + | + | RAF | D-Raffinose** | + | + |
| SAC | White Sugar** | + | + | SOR | D-Sorbitol** | - | - |
| MAL | Maltose** | + | + | RHA | L-Rhamnose** | - | - |
| SAL | Salicin** | + | + | TRE | D-Trehalose** | - | + |
| XYL | D-Xylose** | - | + | | | | |
| ARA | L-Arabinose** | + | + | | | | |
| GEL | Gelatin Hydrolysis* | - | | - | | | |
| ESC | Esculin Hydrolysis* | + | | + | | | |

| | | | | | | | |
|---|---|---|---|---|---|---|---|
| *Biochemistry test,**Oxidation test +: positive, -: negative | | | | | | | |

In addition, additional physiological and biochemical properties of the 01-B1 strain were tested by using an API ZYM kit (bioMerieux, FRA). The results are shown in Table 8-3.

**[Table 8-3]**

| Table 8-3. Physiological and Biochemical Properties Test (Part 3) | |
|---|---|
| Test item | 01-B1 strain |
| Alkaline phosphatase | - |
| Esterase (C4) | +w |
| Esterase lipase (C8) | - |
| Lipase (C14) | - |
| Leucine arylamidase | - |
| Valine arylamidase | - |
| Cystine arylamidase | - |
| Trypsin | - |
| Chymotrypsin | - |
| Acid phosphatase | +w |
| Naphthol-AS-BI-phosphohydrolase | +w |
| α-Galactosidase | +w |
| β-Galactosidase | - |
| β-Glucuronidase | - |
| α-Glucosidase | + |
| β-Glucosidase | - |
| N-acetyl-β-glucosaminidase | - |
| α-Mannosidase | - |
| α-Fucosidase | - |

| | |
|---|---|
| +: Positive, -: Negative, +w: Weak reaction | |

The 01-B1 strain did not have the ability to assimilate D-xylose and D-trehalose (Table 8-2).

On the other hand, the type strain had the ability to assimilate D-xylose and D-trehalose.

Therefore, the 01-B1 strain was determined to be a strain different from the type strain.

### [Example 8: Organic Acid-Producing Capacity Test]

The 01-B1 strain, the B6 strain (test strain 1), and the type strain were cultured anaerobically at 37°C for 72 hours (culture liquid volume: 200 mL) by using a liquid culture medium (AccuDia GAM Broth (Shimadzu Diagnostics Corporation, Japan)) and using AneroPouch Kenki System (Mitsubishi Gas Chemical, Japan) as an anaerobic culture kit.

The culture liquid was filtered through a membrane filter with a pore size of 0.20 µm to obtain a sample solution.

The concentrations of organic acids contained in the sample solution were measured using high-performance liquid chromatography.

The measurement conditions are as follows.
- System: Nexera Organic Acid Analysis System (Shimadzu Corporation)
- Model: LC-40D (Shimadzu Corporation)
- Detector: Electrical conductivity meter CDD-10Avp (Shimadzu Corporation)
- Columns: Shim-pack SCR-102H×2, φ8.0 mm×300 mm (Shimadzu Corporation)
- Card column: SCR-102H, φ6.0 mm×50 mm (Shimadzu Corporation)
- Column temperature: 45°C
- Mobile phase: 5 mmol/L p-toluenesulfonic acid
- Reaction solution: 5 mmol/L p-toluenesulfonic acid containing 0.1 mmol/L EDTA and 20 mmol/L Bis-Tris
- Flow rate: Mobile phase 0.8 mL/min, Reaction solution 0.8 mL/min
- Injection volume: 10 µL

The organic acids measured were four types including lactic acid, formic acid, acetic acid, and n-butyric acid. The results are presented in Table 9.

**[Table 9]**

| Table 9. Organic Acid-Producing Capacity Test (Production Level Unit: g/100g) | | | | |
|---|---|---|---|---|
| Sample name | Lactic acid | Formic acid | Acetic acid | n-Butyric acid |
| Control (culture medium only) | 0.01 | | 0.02 | |
| Type strain (NBRC13949T) | 0.10 | 0.06 | 0.14 | 0.16 |
| B6 strain | 0.10 | | 0.13 | 0.18 |
| 01-B1 strain | 0.13 | | 0.13 | 0.18 |

In the table, a blank cell indicates a value below a lower limit of quantification. The lower limit of quantification was 0.01 g/100 g.

The 01-B1 strain produced n-butyric acid. The level of formic acid production in the culture liquid after 72 hours of anaerobic culture of the 01-B1 strain at 37°C was less than that of the type strain (NBRC13949T).

### [Reference Example 4: Analysis on Partial 16S rDNA Nucleotide Sequence]

A partial nucleotide sequence (SEQ ID NO: 174) of 16S rDNA (16S rRNA gene) of the 01-B1 strain was analyzed to estimate its taxonomic assignment.

[SEQ ID NO: 174]
GACGAACGCTGGCGGCGTGCTTAACACATGCAAGTCGAGCGATGAAGCTCC
TTCGGGAGTGGATTAGCGGCGGACGGGTGAGTAACACGTGGGTAACCTGCCT
CATAGAGGGGAATAGCCTTTCGAAAGGAAGATTAATACCGCATAAGATTGTAG
TACCGCATGGTACAGCAATTAAAGGAGTAATCCGCTATGAGATGGACCCGCG
TCGCATTAGCTAGTTGGTGAGGTAACGGCTCACCAAGGCGACGATGCGTAGC
CGACCTGAGAGGGTGATCGGCCACATTGGGACTGAGACACGGCCCAGACTC
CTACGGGAGGCAGCAGTGGGGAATATTGCACAATGGGGGAAACCCTGATGC
AGCAACGCCGCGTGAGTGATGACGGTCTTCGGATTGTAAAGCTCTGTCTTTA
GGGACGATAATGACGGTACCTAAGGAGGAAGCCACGGCTAACTACGTGCCA
GCAGCCGCGGTAATACGTAGGTGGCAAGCGTTGTCCGGATTTACTGGGCGTA
AAGGGAGCGTAGGTGGATATTTAAGTGGGATGTGAAATACYCGGGCTTAACC
TGGGTGCTGCATTCCAAACTGGATATCTAGAGTGCAGGAGAGGAAAGGAGA
ATTCCTAGTGTAGCGGTGAAATGCGTAGAGATTAGGAAGAATACCAGTGGCG
AAGGCGCCTTTCTGGACTGTAACTGACACTGAGGCTCGAAAGCGTGGGGAG
CAAACAGGATTAGATACCCTGGTAGTCCACGCCGTAAACGATGAATACTAGG
TGTAGGGGTTGTCATGACCTCTGTGCCGCCGCTAACGCATTAAGTATTCCGCC
TGGGGAGTACGGTCGCAAGATTAAAACTCAAAGGAATTGACGGGGGCCCGC
ACAAGCAGCGGAGCATGTGGTTTAATTCGAAGCAACGCGAAGAACCTTACCT
AGACTTGACATCTCCTGAATTACTCTGTAATGGAGGAAGCCACTTCGGTGGC
AGGAAGACAGGTGGTGCATGGTTGTCGTCAGCTCGTGTCGTGAGATGTTGGG
TTAAGTCCCGCAACGAGCGCAACCCTTATTGTTAGTTGCTACCATTTAGTTGA
GCACTCTAGCGAGACTGCCCGGGTTAACCGGGAGGAAGGTGGGGATGACGT
CAAATCATCATGCCCCTTATGTCTAGGGCTACACACGTGCTACAATGGTCGGT
ACAATGAGATGCAACCTCGCGAGAGTGAGCAAAACTATAAAACCGATCTCAG
TTCGGATTGTAGGCTGAAACTCGCCTACATGAAGCTGGAGTTGCTAGTAATCG
CGAATCAGAATGTCGCGGTGAATACGTTCCCGGGCCTTGTACACACCGCCCG
TCACACCATGAGAGTTGGCAATACCCAAAGTTCGTGAGCTAACCGCAAGGA
GGCAGCGACCTAGGTAGGGTCAGCGATTGGGGTGAAG

The analysis was conducted under the following conditions. The results are shown in Tables 10-1 and 10-2.
- DNA extraction: Achromopeptidase (FUJIFILM Wako Pure Chemical, Japan)
- PCR amplification: TKs Gflex DNA Polymerase (Takara Bio, Japan)
- Cycle Sequence: BigDye Terminator v3.1 Cycle Sequencing Kit (Applied Biosystems, USA)
- Used Primers:
   PCR amplification: 9F, 1510R
   Sequencing (approx. 1500 bp): 9F, 515F, 1099F, 536R, 926R, 1510R
- Sequencer: ABI PRISM 3500xL Genetic Analyzer System (Applied Biosystems)
- Nucleotide sequence determination: ChromasPro 2.1 (Technelysium, AUS)
- BLAST homology search:
   Analysis software: ENKI v3.2 (TechnoSuruga Laboratory, Japan)
   Database:
      DB-BA 17.0 (TechnoSuruga Laboratory)
      International DNA sequencing database (DDBJ/ENA/GenBank)
- Simplified molecular phylogenetic tree analysis:
   Phylogenetic tree estimation: Neighbor-joining method
   Nucleotide substitution model: Kimura-2-parameter
   Reliability evaluation of tree structure: Bootstrap method (1,000 repetitions)

**[Table 10-1]**

| Table 10-1 BLAST search results of 01-B1 strain against DB-BA: Homology Rate with Top 30 Nucleotide sequences | | | | | |
|---|---|---|---|---|---|
| Registered Name | Strain Name | Accession No. | Homology Rate | BSL | |
| 1 | *Clostridium butyricum* | JCM1391 | AB595129 | 1437/1438 (99.9%) | |
| 2 | *Clostridium beijerinckii* | NCIMB9362 | X68180 | 1408/1439 (97.8%) | |
| 3 | *Clostridium puniceum* | DSM2619 | X71857 | 1404/1438 (97.6%) | |
| 4 | *Clostridium beijerinckii* | DSM791 | X68179 | 1406/1439 (97.7%) | |
| 5 | *Clostridium chromiireducens* | GCAF-1 | AY228334 | 1400/1438 (97.4%) | |
| 6 | *Clostridium saccharoperbutylacetonicum* | N1-4 | U16122 | 1389/1419 (97.9%) | |
| 7 | *Clostridium saccharobutylicum* | NCP262 | U16147 | 1382/1419 (97.4%) | |
| 8 | *Clostridium neonatale* | CCUG46077 | AF275949 | 1390/1440 (96.5%) | |
| 9 | *Clostridium paraputrificum* | JCM1293 | AB536771 | 1379/1440 (95.8%) | 1* |
| 10 | *Clostridium vincenni* | lac-1 | X97432 | 1368/1440 (95.0%) | |
| 11 | *Clostridium charrarabidum* | DSM5482 | X71850 | 1365/1437 (95.0%) | |
| 12 | *Clostridium uliginosum* | CK55 | AJ276992 | 1355/1422 (95.3%) | |
| 13 | *Clostridium saudiense* | JCC | HG726039 | 1364/1442 (94.6%) | |
| 14 | *Clostridium sardiniense* | DSM2632 | AB161367 | 1364/1442 (94.6%) | |
| 15 | *Clostridium tertium* | JCM6289 | AB618789 | 1363/1442 (94.5%) | 1* |
| 16 | *Clostridium disporicum* | DSM5521 | Y18176 | 1364/1443 (94.5%) | 1* |
| 17 | *Clostridium sortagoforme* | DSM1292 | Y18175 | 1361/1442 (94.4%) | |
| 18 | *Clostridium colicanis* | DSM13634 | AJ420008 | 1356/1436 (94.4%) | |
| 19 | *Clostridium bararii* | ATCC27638 | X68174 | 1358/1442 (94.2%) | 1* |
| 20 | *Clostridium gasigenes* | DSM12272 | AF092548 | 1358/1442 (94.2%) | |
| 21 | *Clostridium chauvoei* | ATCC10092 | U51843 | 1355/1441 (94.0%) | 2 |
| 22 | *Clostridium quinii* | DSM6736 | X76745 | 1360/1443 (94.2%) | |
| 23 | *Clostridium moniliforme* | JCM9990 | AB540985 | 1357/1442 (94.1%) | 1* |
| 24 | *Clostridium tarantellae* | DSM3997 | FR733677 | 1351/1438 (93.9%) | 1* |
| 25 | *Clostridium septicum* | ATCC12464 | U59278 | 1351/1441 (93.8%) | 2 |
| 26 | *Clostridium budayi* | ATCC25541 | AB018183 | 1349/1441 (93.6%) | |
| 27 | *Clostridium algidicarnis* | NCFB2931 | AF127023 | 1344/1441 (93.3%) | |
| 28 | *Inconstantimicrobium porci* | WCA-383-APC-5B | MN537551 | 1343/1442 (93.1%) | |
| 29 | *Clostridium putrefaciens* | DSM1291 | AF127024 | 1343/1441 (93.2%) | |
| 30 | *Clostridium cavendishii* | BL-28 | DQ196621 | 11331/1424 (93.5%) | |

| | | | | | |
|---|---|---|---|---|---|
| Note 1) In BSL (Bio Safety Level), Level 1* (Opportunistic pathogens) or higher is written and a blank cell means Level 1. Note 2) The underscore "_" in the strain name represents a space. Note 3) The 11 strains, that is, the 1st to 3rd and 5th to 12th strains from the top, in Table represent sequence data used for simplified molecular phylogenetic analysis. | | | | | |

**[Table 10-2]**

| Table 10-2 BLAST Search Results of 01-B1 Strain against International DNA Sequencing Database: Homology Rate with Top 30 Nucleotide Sequences | | | |
|---|---|---|---|
| Registered Name | Strain Name | Accession No. | Homology Rate |
| Clostridium butyricum | 16-3 | CP053292 | 1437/1438 (99.9%) |
| Clostridium butyricum | CFSA3989 | CP033249 | 1437/1438 (99.9%) |
| Clostridium butyricum | CFSA3987 | CP033247 | 1437/1438 (99.9%) |
| Clostridium butyricum | NBRC 13949 | AP019716 | 1437/1438 (99.9%) |
| Clostridium butyricum | 4-1 | CP039705 | 1437/1438 (99.9%) |
| Clostridium butyricum | MSK | MK571204 | 1437/1438 (99.9%) |
| Clostridium butyricum | TMN60 | LC784037 | 1438/1438 (100.0%) |
| Clostridium butyricum | TMN59 | LC784036 | 1437/1438 (99.9%) |
| Clostridium butyricum | TK520 | CP016332 | 1437/1438 (99.9%) |
| Clostridium butyricum | CBM588 | CP132344 | 1437/1438 (99.9%) |
| Clostridium butyricum | TOA | CP014704 | 1437/1438 (99.9%) |
| Clostridium butyricum | DKU-11 | CP128832 | 1437/1438 (99.9%) |
| Clostridium butyricum | SJ1 | CP127838 | 1437/1438 (99.9%) |
| Clostridium butyricum | JKY6D1 | CP013352 | 1437/1438 (99.9%) |
| Clostridium butyricum | KNU-L09 | CP013252 | 1437/1438 (99.9%) |
| Clostridium butyricum | LVI | CP114763 | 1437/1438 (99.9%) |
| Clostridium butyricum | NEC4 | LN828931 | 1437/1438 (99.9%) |
| Clostridium butyricum | QXYZ514 | CP049778 | 1437/1438 (99.9%) |
| Clostridium sp. | LQ25 | CP111077 | 1437/1438 (99.9%) |
| Clostridium butyricum | CD-M-8 | OP491983 | 1437/1438 (99.9%) |
| Clostridium butyricum | CFSA-TJ-E | CP053374 | 1437/1438 (99.9%) |
| Clostridium butyricum | BUT1 | ON142182 | 1437/1438 (99.9%) |
| Clostridium sp. | BG-M-101 | OK271747 | 1437/1438 (99.9%) |
| Clostridium sp. | B2-M-100 | OK271596 | 1437/1438 (99.9%) |
| Clostridium butyricum | CBUT | CP073277 | 1437/1438 (99.9%) |
| Clostridium butyricum | DS501 | CP071447 | 1437/1438 (99.9%) |
| Clostridium butyricum | CB1 | MW447605 | 1437/1438 (99.9%) |
| Clostridium butyricum | CB TO-A | AB687551 | 1437/1438 (99.9%) |
| Clostridium butyricum | DSM 10702 | CP040626 | 1437/1438 (99.9%) |
| Clostridium butyricum | JCM 1391 | NR 113244 | 1437/1438 (99.9%) |

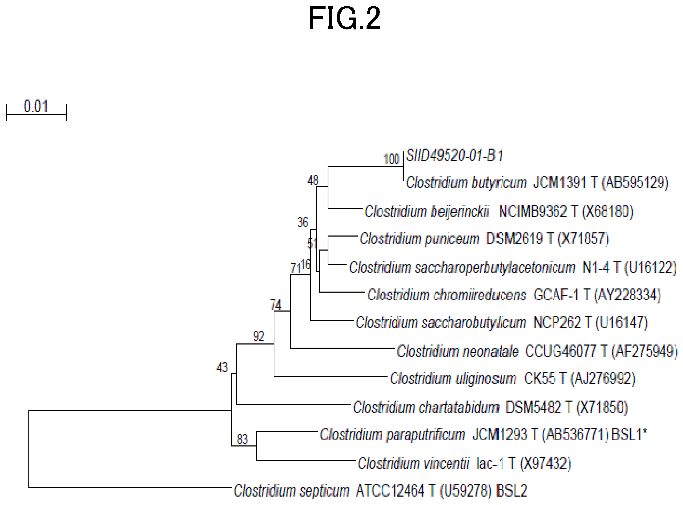

Fig. 2 shows a simplified molecular phylogenetic tree of the 01-B1 strain based on the partial 16S rDNA nucleotide sequences. In Fig. 2, SIID49520-01-B1 represents the 01-B1 strain. A line on the left upper side shows a scale bar. The number located at each branching point of phylogenetic branches represents a bootstrap value, which is a values indicating the reliability of the tree structure. The suffix T of the strain name indicates the type strain (Type) of that species. BSL indicates the biosafety level (BSL1* (opportunistic pathogen) or higher).

### [Reference to Deposited Biological Material]

Name of depositary authority: NITE Patent Microorganisms Depositary
Contact address: Room 122, 2-5-8 Kazusa-Kamatari, Kisarazu City, Chiba Prefecture, Japan 292-0818
Accession No: NITE BP-04190
Indication of identification: SIID49520-01-B1
Deposit date: October 29, 2024
Origin: Japan
Source of isolation: Isolated from a fermentation liquid containing a culture medium fermented at the food raw material manufacturing plant of HigherMount Co., Ltd. in Kaizu City, Gifu Prefecture. As the culture medium, domestically produced soybeans were used. The soybeans used were organically grown in Japan without use of pesticides.

### [Test Strain 3]

The *Clostridium butyricum* SIID 50030-B1 strain (hereinafter also referred to as "the B1 strain") was used.

The B1 strain is internationally deposited with NITE Patent Microorganisms Depositary, International Depositary Authority under the provisions of the Budapest Convention (Room 122, 2-5-8 Kazusa-Kamatari, Kisarazu City, Chiba Prefecture, Japan 292-0818) (Indication of identification: SIID 50030-B1. Accession number: NITE BP-04191. Deposit date (Accession date): October 29, 2024).

### [Example 9: Morphological, Physiological, And Biochemical Properties Test]

The B1 strain was cultured anaerobically at 37°C for 24 hours by using an agar culture medium (AccuDia GAM Broth (Shimadzu Diagnostics Corporation, Japan)+agar) and using AneroPouch Kenki System (Mitsubishi Gas Chemical, Japan) as an anaerobic culture kit.

The morphology of the cultured B1 strain was observed by using an optical microscope.

Moreover, the physiological properties of the B1 strain (catalase reaction, oxidase reaction, acid/gas production from glucose, and oxidation/fermentation (O/F) of glucose) were tested based on the method of Barrow & Feltham et al. (Cowan and Steel's Manual for the Identification of Medical Bacteria. 3rd edition. Cambridge: University Press; 1993). The results are shown in Table 11-1.

The results of the type strain in Table 11-1 were obtained in Example 1.

**[Table 11-1]**

| Table 11-1. Morphological Observation and Physiological Properties Tests (Part 1) | | | |
|---|---|---|---|
| Test item | | B1 strain | Type strain |
| Culture temperature | | 37°C | 37°C |
| Cell morphology | | Rod-shaped bacterium (0.9-1.1×2.0-8.0 µm) | Rod-shaped bacterium (0.7-0.9×2.0-8.0 µm) |
| Gram stainability | | + | + |
| Presence or absence of spores | | + | + (Central/Subterminal) |
| Motility | | - | + |
| Colony form | Culture medium | GAM broth agar culture medium | GAM agar culture medium |
| | Culture time | 24 hours | 24 hours |
| | Diameter | 1-4 mm | N/R |
| | Tone | Pale yellow | Pale yellow |
| | Shape | Irregular shapes | N/R |
| | Elevation | Lens-shaped | N/R |
| | Margin | Undulate | Undulate |
| | Surface shape, etc. | Smooth | N/R |
| | Transparency | Opaque | N/R |
| | Consistency | Butter-like | N/R |
| Growth temperature test | 30°C | + | + |
| | 45°C | + | - |
| Catalase reaction | | - | - |
| Oxidase reaction | - | - | |
| Acid/gas production from glucose (Acid production/Gas generation) | -/- | -/N/R | |
| O/F test (Oxidation/Fermentation) | -/- | N/R | |

| | | | |
|---|---|---|---|
| +: Positive, -: Negative, +w: Weak reaction, N/R: Not measured | | | |

The B1 strain was cultured anaerobically at 37°C for 24 hours by using an agar culture medium (AccuDia GAM Broth (Shimadzu Diagnostics Corporation, Japan)+agar) and using AneroPouch Kenki System (Mitsubishi Gas Chemical, Japan) as an anaerobic culture kit.

The physiological and biochemical properties of the cultured B1 strain were identified by using an anaerobic bacterial biochemical identification kit (API20A (bioMerieux, FRA)). The results are shown in Table 11-2.

The results of the type strain in Table 11-2 were obtained in Example 1.

**[Table 11-2]**

| Table 11-2. Physiological and Biochemical Properties Test (Part 2) | | | | | | | |
|---|---|---|---|---|---|---|---|
| Test item | Reaction/Enzyme | B1 strain | Type strain | Test item | Reaction/Enzyme | B1 strain | Type strain |
| IND | Indol Production* | - | - | GLY | Glycerin** | - | - |
| URE | Urease* | - | - | CEL | D-Cellobiose** | + | + |
| GLU | Glucose** | + | + | MNE | D-Mannose** | + | + |
| MAN | D-Mannitol** | - | - | MLZ | D-Melezitose** | - | + |
| LAC | Lactose** | + | + | RAF | D-Raffinose** | + | + |
| SAC | White Sugar** | + | + | SOR | D-Sorbitol** | - | - |
| MAL | Maltose** | + | + | RHA | L-Rhamnose** | - | - |
| SAL | Salicin** | + | + | TRE | D-Trehalose** | - | + |
| XYL | D-Xylose** | - | + | | | | |
| ARA | L-Arabinose** | + | + | | | | |
| GEL | Gelatin Hydrolysis* | - | - | | | | |
| ESC | Esculin Hydrolysis* | + | + | | | | |

| | | | | | | | |
|---|---|---|---|---|---|---|---|
| *Biochemistry test,**Oxidation test +: positive, -: negative | | | | | | | |

In addition, additional physiological and biochemical properties of the B1 strain were tested by using an API ZYM kit (bioMerieux, FRA). The results are shown in Table 11-3.

**[Table 11-3]**

| Table 11-3. Physiological and Biochemical Properties Test (Part 3) | |
|---|---|
| Test item | B1 strain |
| Alkaline phosphatase | - |
| Esterase (C4) | +w |
| Esterase lipase (C8) | - |
| Lipase (C14) | - |
| Leucine arylamidase | - |
| Valine arylamidase | - |
| Cystine arylamidase | - |
| Trypsin | - |
| Chymotrypsin | - |
| Acid phosphatase | +w |
| Naphthol-AS-BI-phosphohydrolase | +w |
| α-Galactosidase | +w |
| β-Galactosidase | - |
| β-Glucuronidase | - |
| α-Glucosidase | + |
| β-Glucosidase | - |
| N-acetyl-β-glucosaminidase | - |
| α-Mannosidase | - |
| α-Fucosidase | - |

| | |
|---|---|
| +: Positive, -: Negative, +w: Weak reaction | |

The B1 strain did not have the ability to assimilate D-xylose and D-trehalose (Table 11-2).

On the other hand, the type strain had the ability to assimilate D-xylose and D-trehalose.

Therefore, the B1 strain was determined to be a strain different from the type strain.

### [Example 10: Organic Acid-Producing Capacity Test]

The B1 strain, the B6 strain (test strain 1), and the type strain were cultured anaerobically at 37°C for 72 hours (culture liquid volume: 200 mL) by using a liquid culture medium (AccuDia GAM Broth (Shimadzu Diagnostics Corporation, Japan)) and using AneroPouch Kenki System (Mitsubishi Gas Chemical, Japan) as an anaerobic culture kit.

The culture liquid was filtered through a membrane filter with a pore size of 0.20 µm to obtain a sample solution.

The concentrations of organic acids contained in the sample solution were measured using high-performance liquid chromatography.

The measurement conditions are as follows.
- System: Nexera Organic Acid Analysis System (Shimadzu Corporation)
- Model: LC-40D (Shimadzu Corporation)
- Detector: Electrical conductivity meter CDD-10Avp (Shimadzu Corporation)
- Column: Shim-pack SCR-102H×2, φ8.0 mm×300 mm (Shimadzu Corporation)
- Card column: SCR-102H, φ6.0 mm×50 mm (Shimadzu Corporation)
- Column temperature: 45°C
- Mobile phase: 5 mmol/L p-toluenesulfonic acid
- Reaction solution: 5 mmol/L p-toluenesulfonic acid containing 0.1 mmol/L EDTA and 20 mmol/L Bis-Tris
- Flow rate: Mobile phase 0.8 mL/min, Reaction solution 0.8 mL/min
- Injection volume: 10 µL

The organic acids measured were four types including lactic acid, formic acid, acetic acid, and n-butyric acid. The results are shown in Table 12.

**[Table 12]**

| Table 12. Organic Acid-Producing Capacity Test (Production Level Unit: g/100g) | | | | |
|---|---|---|---|---|
| Sample name | Lactic acid | Formic acid | Acetic acid | n-Butyric acid |
| Control (culture medium only) | 0.01 | | 0.02 | |
| Type strain (NBRC13949T) | 0.10 | 0.06 | 0.14 | 0.16 |
| B6 strain | 0.10 | | 0.13 | 0.18 |
| B1 strain | 0.10 | | 0.13 | 0.19 |

In the table, a blank cell indicates a value below a lower limit of quantification. The lower limit of quantification was 0.01 g/100 g.

The B1 strain produced n-butyric acid. The level of formic acid production in the culture liquid after 72 hours of anaerobic culture of the B1 strain at 37°C was less than that of the type strain (NBRC13949T).

### [Reference Example 5: Analysis on Partial 16S rDNA nucleotide sequence]

A partial nucleotide sequence (SEQ ID NO: 175) of 16S rDNA (16S rRNA gene) of the B1 strain was analyzed to estimate its taxonomic assignment.

[SEQ ID NO: 175]
GACGAACGCTGGCGGCGTGCTTAACACATGCAAGTCGAGCGATGAAGCTCC
TTCGGGAGTGGATTAGCGGCGGACGGGTGAGTAACACGTGGGTAACCTGCCT
CATAGAGGGGAATAGCCTTTCGAAAGGAAGATTAATACCGCATAAGATTGTAG
TACCGCATGGTACAGCAATTAAAGGAGTAATCCGCTATGAGATGGACCCGCG
TCGCATTAGCTAGTTGGTGAGGTAACGGCTCACCAAGGCGACGATGCGTAGC
CGACCTGAGAGGGTGATCGGCCACATTGGGACTGAGACACGGCCCAGACTC
CTACGGGAGGCAGCAGTGGGGAATATTGCACAATGGGGGAAACCCTGATGC
AGCAACGCCGCGTGAGTGATGACGGTCTTCGGATTGTAAAGCTCTGTCTTTA
GGGACGATAATGACGGTACCTAAGGAGGAAGCCACGGCTAACTACGTGCCA
GCAGCCGCGGTAATACGTAGGTGGCAAGCGTTGTCCGGATTTACTGGGCGTA
AAGGGAGCGTAGGTGGATATTTAAGTGGGATGTGAAATACYCGGGCTTAACC
TGGGTGCTGCATTCCAAACTGGATATCTAGAGTGCAGGAGAGGAAAGGAGA
ATTCCTAGTGTAGCGGTGAAATGCGTAGAGATTAGGAAGAATACCAGTGGCG
AAGGCGCCTTTCTGGACTGTAACTGACACTGAGGCTCGAAAGCGTGGGGAG
CAAACAGGATTAGATACCCTGGTAGTCCACGCCGTAAACGATGAATACTAGG
TGTAGGGGTTGTCATGACCTCTGTGCCGCCGCTAACGCATTAAGTATTCCGCC
TGGGGAGTACGGTCGCAAGATTAAAACTCAAAGGAATTGACGGGGGCCCGC
ACAAGCAGCGGAGCATGTGGTTTAATTCGAAGCAACGCGAAGAACCTTACCT
AGACTTGACATCTCCTGAATTACTCTGTAATGGAGGAAGCCACTTCGGTGGC
AGGAAGACAGGTGGTGCATGGTTGTCGTCAGCTCGTGTCGTGAGATGTTGGG
TTAAGTCCCGCAACGAGCGCAACCCTTATTGTTAGTTGCTACCATTTAGTTGA
GCACTCTAGCGAGACTGCCCGGGTTAACCGGGAGGAAGGTGGGGATGACGT
CAAATCATCATGCCCCTTATGTCTAGGGCTACACACGTGCTACAATGGTCGGT
ACAATGAGATGCAACCTCGCGAGAGTGAGCAAAACTATAAAACCGATCTCAG
TTCGGATTGTAGGCTGAAACTCGCCTACATGAAGCTGGAGTTGCTAGTAATCG

CGAATCAGAATGTCGCGGTGAATACGTTCCCGGGCCTTGTACACACCGCCCG
TCACACCATGAGAGTTGGCAATACCCAAAGTTCGTGAGCTAACCGCAAGGA
GGCAGCGACCTAAGGTAGGGTCAGCGATTGGGGTGAAG

The analysis was conducted under the following conditions. The results are shown in Tables 13-1 and 13-2.
- DNA extraction: Achromopeptidase (FUJIFILM Wako Pure Chemical, Japan)
- PCR amplification: TKs Gflex DNA Polymerase (Takara Bio, Japan)
- Cycle Sequencing: BigDye Terminator v3.1 Cycle Sequencing Kit (Applied Biosystems, USA)
- Used Primers:
   PCR amplification: 9F, 1510R
   Sequencing (approx. 1500 bp): 9F, 515F, 1099F, 536R, 802R, 1510R
- Sequencer: ABI PRISM 3500xL Genetic Analyzer System (Applied Biosystems)
- Nucleotide sequence determination: ChromasPro 2.1 (Technelysium, AUS)
- BLAST homology search:
   Analysis software: ENKI v3.2 (TechnoSuruga Laboratory, Japan)
   Database:
      DB-BA 17.0 (TechnoSuruga Laboratory)
      International DNA sequencing database (DDBJ/ENA/GenBank)
- Simplified molecular phylogenetic tree analysis:
   Phylogenetic tree estimation: Neighbor-joining method
   Nucleotide substitution model: Kimura-2- parameter
   Reliability evaluation of tree structure: Bootstrap method (1,000 repetitions)

**[Table 13-1]**

| Table 13-1 BLAST Search Results of B1 Strain against DB-BA: Homology Rate With Top 30 Nucleotide Sequences | | | | | |
|---|---|---|---|---|---|
| Registered Name | Strain Name | Accession | Homology No. | BSL Rate | |
| **1** | ***Clostridium butyricum*** | **JCM1391** | **AB595129** | **1437/1438 (99.9%)** | |
| **2** | ***Clostridium beijerinckii*** | **NCIMB9362** | **X68180** | **1408/1439 (97.8%)** | |
| **3** | ***Clostridium puniceum*** | **DSM2619** | **X71857** | **1404/1438 (97.6%)** | |
| **4** | ***Clostridium beijerinckii*** | **DSM791** | **X68179** | **1406/1439 (97.7%)** | |
| **5** | ***Clostridium chromiireducens*** | **GCAF-1** | **AY228334** | **1400/1438 (97.4%)** | |
| **6** | ***Clostridium saccharoperburylacetonicum*** | **N1-4** | **U16122** | **1389/1419 (97.9%)** | |
| **7** | ***Clostridium saccharobutylicum*** | **NCP262** | **U16147** | **1382/1419 (97.4%)** | |
| **8** | ***Clostridium neonatale*** | **CCUG46077** | **AF275949** | **1390/1440 (96.5%)** | |
| **5** | ***Clostridium paraputrificum*** | **JCM1293** | **AB536771** | **1379/1440 (95.8%)** | **1*** |
| **10** | ***Clostridium vincentii*** | **lac-1** | **X97432** | **1368/1440 (95.0%)** | |
| **11** | ***Clostridium chartatabidum*** | **DSM5482** | **X71850** | **1365/1437 (95.0%)** | |
| **12** | ***Clostridium uliginosum*** | **CK55** | **AJ276992** | **1355/1422 (95.3%)** | |
| **13** | ***Clostridium saudiense*** | **JCC** | **HG726039** | **1364/1442 (94.6%)** | |
| **14** | ***Clostridium sardiniense*** | **DSM2632** | **AB161367** | **1364/1442 (94.6%)** | |
| **15** | ***Clostridium tertium*** | **JCM6289** | **AB618789** | **1363/1442 (94.5%)** | **1*** |
| **16** | ***Clostridium disporicum*** | **DSM5521** | **Y18176** | **1364/1443 (94.5%)** | **1*** |
| **17** | ***Clostridium sartagoforme*** | **DSM1292** | **Y18175** | **1361/1442 (94.4%)** | |
| **18** | ***Clostridium colicanis*** | **DSM13634** | **AJ420008** | **1356/1436 (94.4%)** | |
| **19** | ***Clostridium baratti*** | **ATCC27638** | **X68174** | **1358/1442 (94.2%)** | **1*** |
| **20** | ***Clostridium gasigenes*** | **DSM12272** | **AF092548** | **1358/1442 (94.2%)** | |
| **21** | ***Clostridium chauvoei*** | **ATCC10092** | **U51843** | **1355/1441 (94.0%)** | **2** |
| **22** | ***Clostridium quinii*** | **DSM6736** | **X76745** | **1360/1443 (94.2%)** | |
| **23** | ***Clostridium moniliforme*** | **JCM9990** | **AB540985** | **1357/1442 (94.1%)** | **1*** |
| **24** | ***Clostridium tarantellae*** | **DSM3997** | **FR733677** | **1351/1438 (93.9%)** | **1*** |
| **25** | ***Clostridium septicum*** | **ATCC12464** | **U59278** | **1351/1441 (93.8%)** | **2** |
| **26** | ***Clostridium budayi*** | **ATCC25541** | **AB018183** | **1349/1441 (93.6%)** | |
| **27** | ***Clostridium algidicarnis*** | **NCFB2931** | **AF127023** | **1344/1441 (93.3%)** | |
| **28** | ***Inconstantimicrobium porci*** | **WCA-383-APC-5B** | **MN537551** | **1343/1442 (93.1%)** | |
| **29** | ***Clostridium putrefaciens*** | **DSM1291** | **AF127024** | **1343/1441 (93.2%)** | |
| **30** | ***Clostridium cavendishii*** | **BL-28** | **DQ196621** | **1331/1424 (93.5%)** | |

| | | | | | |
|---|---|---|---|---|---|
| Note 1) In BSL (Bio Safety Level), Level 1* (Opportunistic pathogens) or higher is written and a blank cell means Level 1. Note 2) The underscore "_" in the strain name represents a space. Note 3) The 10 strains, that is, the 1st to 3rd and 5th to 11th strains from the top, in Table represent sequence data used for simplified molecular phylogenetic analysis. | | | | | |

**[Table 13-2]**

| Table 13-2 BLAST Search Results Of B1 Strain against International DNA Sequencing Database: Homology Rate with Top 30 Nucleotide Sequences | | | |
|---|---|---|---|
| Registered Name | Strain Name | Accession No. | Homology Rate |
| Clostridium butyricum | 16-3 | CP053292 | 1437/1438 (99.9%) |
| Clostridium butyricum | CFSA3989 | CP033249 | 1437/1438 (99.9%) |
| Clostridium butyricum | CFSA3987 | CP033247 | 1437/1438 (99.9%) |
| Clostridium butyricum | NBRC 13949 | AP019716 | 1437/1438 (99.9%) |
| Clostridium butyricum | 4-1 | CP039705 | 1437/1438 (99.9%) |
| Clostridium butyricum | MSK | MK571204 | 1437/1438 (99.9%) |
| Clostridium butyricum | TMN60 | LC784037 | 1438/1438 (100.0%) |
| Clostridium butyricum | TMN59 | LC784036 | 1437/1438 (99.9%) |
| Clostridium butyricum | TK520 | CP016332 | 1437/1438 (99.9%) |
| Clostridium butyricum | CBM588 | CP132344 | 1437/1438 (99.9%) |
| Clostridium butyricum | TOA | CP014704 | 1437/1438 (99.9%) |
| Clostridium butyricum | DKU-11 | CP128832 | 1437/1438 (99.9%) |
| Clostridium butyricum | SJ1 | CP127838 | 1437/1438 (99.9%) |
| Clostridium butyricum | JKY6D1 | CP013352 | 1437/1438 (99.9%) |
| Clostridium butyricum | KNU-L09 | CP013252 | 1437/1438 (99.9%) |
| Clostridium butyricum | LV1 | CP114763 | 1437/1438 (99.9%) |
| Clostridium butyricum | NEC4 | LN828931 | 1437/1438 (99.9%) |
| Clostridium butyricum | QXYZ514 | CP049778 | 1437/1438 (99.9%) |
| Clostridium sp. | LQ25 | CP111077 | 1437/1438 (99.9%) |
| Clostridium butyricum | CD-M-8 | OP491983 | 1437/1438 (99.9%) |
| Clostndium butyricum | CFSA-TJ-E | CP053374 | 1437/1438 (99.9%) |
| Clostridium butyricum | BUT1 | ON142182 | 1437/1438 (99.9%) |
| Clostridium sp. | BG-M-101 | OK271747 | 1437/1438 (99.9%) |
| Clostridium sp. | B2-M-100 | OK271596 | 1437/1438 (99.9%) |
| Clostridium butyricum | CBUT | CP073277 | 1437/1438 (99.9%) |
| Clostridium butyricum | DS501 | CP071447 | 1437/1438 (99.9%) |
| Clostridium butyricum | CB1 | MW447605 | 1437/1438 (99.9%) |
| Clostridium butyricum | CB TO-A | AB687551 | 1437/1438 (99.9%) |
| Clostridium butyricum | DSM 10702 | CP040626 | 1437/1438 (99.9%) |
| Clostridium butyricum | JCM 1391 | NR 113244 | 1437/1438 (99.9%) |

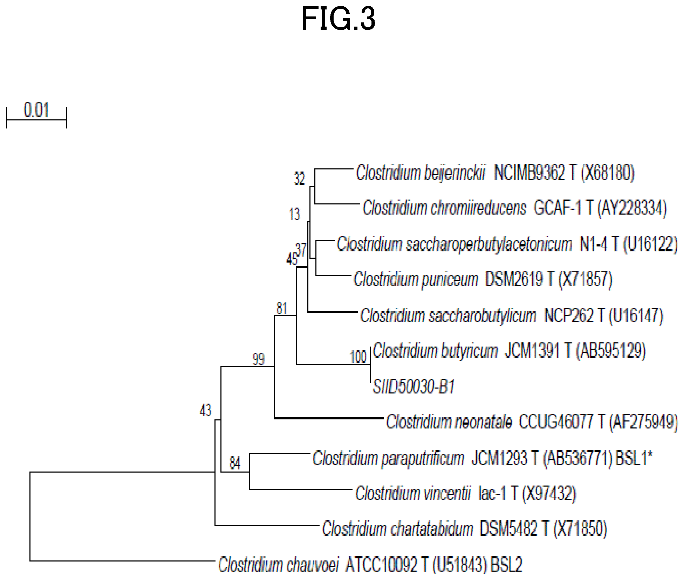

Fig. 3 shows a simplified molecular phylogenetic tree of the B1 strain based on the partial 16S rDNAnucleotide sequences. In Fig. 3, SIID 50030-B1 represents the B1 strain. A line on the left upper side shows a scale bar. The number located at each branching point of phylogenetic branches represents a bootstrap value, which is a values indicating the reliability of the tree structure. The suffix T of the strain name indicates the type strain (Type) of that species. BSL indicates the biosafety level (BSL1* (opportunistic pathogen) or higher).

### [Reference to Deposited Biological Material]

Name of depositary authority: NITE Patent Microorganisms Depositary
Contact address: Room 122, 2-5-8 Kazusa-Kamatari, Kisarazu City, Chiba Prefecture, Japan 292-0818
Accession No: NITE BP-04191
Indication of identification: SIID 50030-B1
Deposit date: October 29, 2024
Origin: Japan
Source of isolation: Isolated from a fermentation liquid containing a culture medium fermented at the food raw material manufacturing plant of Higher Mount Co., Ltd. in Kaizu City, Gifu Prefecture. As the culture medium, domestically produced soybeans were used. The soybeans used were organically grown in Japan without use of pesticides.

Here, the 01-B1 strain (test strain 2) and the B1 strain (test strain 3) were different in the following points. Therefore, the 01-B1 strain was determined to be a strain different from the B1 strain.

| | 01-B1 strain | B1 strain |
|---|---|---|
| Cell morphology | Rod-shaped bacterium (0.9-1.1×2.0-5.0 µm) | Rod-shaped bacterium (0.9-1.1×2.0-8.0 µm) |
| Colony diameter | 2-5 mm | 1-4 mm |
| Lactic acid production level | 0.13 g/100 g | 0.10 g/100 g |
| n-Butyric acid production level | 0.18 g/100 g | 0.19 g/100 g |

### Industrial Applicability

The present invention is usable in foods and beverages, pharmaceuticals, and others.

## Claims

1. *Clostridium butyricum* that produces n-butyric acid and has all of the following features (1) to (3):
(1) have no ability to assimilate D-xylose;
(2) have no ability to assimilate D-trehalose; and
(3) produce a lower level of formic acid in a culture liquid after 72 hours of anaerobic culture at 37°C than a NBRC13949T strain as a type strain of *Clostridium butyricum*.

2. A *Clostridium butyricum* SIID29215-B6 strain (Accession number: NITE BP-03916) or a mutant strain thereof, wherein the mutant strain produces n-butyric acid and has all of the following features (1) to (3):
(1) have no ability to assimilate D-xylose;
(2) have no ability to assimilate D-trehalose; and
(3) produce a lower level of formic acid in a culture liquid after 72 hours of anaerobic culture at 37°C than a NBRC13949T strain as a type strain of *Clostridium butyricum*.

3. A *Clostridium butyricum* SIID29215-B6 strain (Accession number: NITE BP-03916).

4. A *Clostridium butyricum* SIID49520-01-B1 strain (Accession number: NITE BP-04190) or a mutant strain thereof, wherein the mutant strain produces n-butyric acid and has all of the following features (1) to (3):
(1) have no ability to assimilate D-xylose;
(2) have no ability to assimilate D-trehalose; and
(3) produce a lower level of formic acid in a culture liquid after 72 hours of anaerobic culture at 37°C than a NBRC13949T strain as a type strain of *Clostridium butyricum*.

5. A *Clostridium butyricum* SIID49520-01-B1 strain (Accession number: NITE BP-04190).

6. A *Clostridium butyricum* SIID 50030-B1 strain (Accession number: NITE BP-04191) or a mutant strain thereof, wherein the mutant strain produces n-butyric acid and has all of the following features (1) to (3):
(1) have no ability to assimilate D-xylose;
(2) have no ability to assimilate D-trehalose; and
(3) produce a lower level of formic acid in a culture liquid after 72 hours of anaerobic culture at 37°C than a NBRC13949T strain as a type strain of *Clostridium butyricum*.

7. A *Clostridium butyricum* SIID 50030-B1 strain (Accession number: NITE BP-04191).

8. A composition for intestinal regulation comprising, as an active ingredient, bacterial cells, spores, or a culture liquid of the *Clostridium butyricum* according to any one of claims 1 to 7.

9. A composition for growth inhibition of *Clostridioides difficile* comprising, as an active ingredient, bacterial cells, spores, or a culture liquid of the *Clostridium butyricum* according to any one of claims 1 to 7.

10. A composition for anti-inflammatory use comprising, as an active ingredient, bacterial cells, spores, or a culture liquid of the *Clostridium butyricum* according to any one of claims 1 to 7.

11. A composition for induction of regulatory T cells comprising, as an active ingredient, bacterial cells, spores, or a culture liquid of the *Clostridium butyricum* according to any one of claims 1 to 7.

12. The composition according to claim 8, which is in the form of a pharmaceutical product.

13. The composition according to claim 9, which is in the form of a pharmaceutical product.

14. The composition according to claim 10, which is in the form of a pharmaceutical product.

15. The composition according to claim 11, which is in the form of a pharmaceutical product.

16. The composition according to claim 8, which is in the form of a food or beverage product.

17. The composition according to claim 9, which is in the form of a food or beverage product.

18. The composition according to claim 10, which is in the form of a food or beverage product.

19. The composition according to claim 11, which is in the form of a food or beverage product.
